# EUROPEAN PATENT APPLICATION

(11) **EP 4 796 098 A1**
(43) Date of publication of application: **26.08.2026**
(21) Application number: 24903942.1
(22) Date of filing: 22.10.2024
(51) Int. Cl.: A61J 7/04, G16H 20/10, G16H 10/20, A61B 5/024, A61B 5/01, A61B 5/0245, A61B 5/346, A61B 5/00

(54) **ELECTRONIC DEVICE, AND METHOD FOR OUTPUTTING NOTIFICATION SIGNAL FOR MEDICATION INTAKE BY USING SAME**

(30) Priority: 11.12.2023 KR 20230179038; 02.01.2024 KR 20240000482; 25.01.2024 KR 20240011434
(71) Applicant: Samsung Electronics Co., Ltd., Suwon-si, Gyeonggi-do 16677 (KR)
(72) Inventor: LEE, Sehee, Suwon-si, Gyeonggi-do 16677 (KR); LEE, Heonwoo, Suwon-si, Gyeonggi-do 16677 (KR)
(74) Representative: Nederlandsch Octrooibureau
(86) International application number: PCT/KR2024/016105
(87) International publication number: WO 2025/127375

(57) **Abstract**

According to an embodiment, the electronic device may comprise a display, a processor, and a memory for storing a medication intake schedule, and instructions. The instructions, when executed by the processor, may cause the electronic device to: display a clock screen including an icon for at least one medication on the basis of the medication intake schedule; identify a notification time for the intake of the at least one medication on the basis of the medication intake schedule; identify an adjustment condition for a medication intake-related notification signal on the basis of the identified notification time; adjust an output time point of the notification signal for the intake of the at least one medication, on the basis of the identified adjustment condition of the medication intake-related notification signal; and display a visual effect corresponding to the icon included in the clock screen, on the basis of the adjusted output time point. Various other embodiments may be possible.

## Description

### [Technical Field]

Embodiments of the disclosure relate to an electronic device and a method for outputting a notification signal for medication intake using the same.

### [Background Art]

With recent technological advancements, electronic devices are evolving from uniform rectangular shapes into increasingly diverse forms. For example, to enhance user convenience, electronic devices are evolving into wearable electronic devices that can be worn on a part of the body. Such wearable electronic devices may include a wearable watch that can be worn on a user's wrist.

Currently, with growing interest in healthcare, users desire to take various medications at designated times. To ensure the correct intake of various medications, methods are being discussed for sharing information and precautions related to medication intake and providing medication intake-related notifications at scheduled times.

The above information may be presented as related art only to assist with an understanding of the disclosure. No determination has been made, and no assertion is made, as to whether any of the above might be applicable as prior art with regard to the disclosure.

### [Disclosure of Invention]

### [Technical Problem]

Users taking various types of medication may desire to accurately confirm the intake timing and dosage for each medication. However, due to personal activities and schedules, proper medication intake may be difficult to achieve. For example, a problem may arise where medication is not taken correctly at a designated time. Furthermore, if a notification time for medication passes due to other schedules overlapping with the medication schedule, a problem may occur where the user forgets to take the corresponding medication.

An electronic device (e.g., a wearable electronic device or a wearable watch), while being worn on a part of a user's body (e.g., a wrist), may provide the user with notification information regarding the medication to be taken (e.g., medication intake) based on a medication intake schedule. For example, the electronic device may display a user interface related to medication intake through a display. The electronic device may display at least one medication icon for intake and provide a visual effect for the medication icon in accordance with a set medication intake timing. The electronic device may provide the user with a notification signal that stimulates at least one of the user's five senses (e.g., sight, hearing, smell, taste, and touch).

According to an embodiment, there may be provided an electronic device that provides a notification based on a medication intake management application (e.g., a program related to medication intake) to enable a user to correctly take medication in accordance with a set medication intake time.

The technical problems to be solved by the disclosure are not limited to those mentioned above, and other technical problems not mentioned will be clearly understood from the following description by a person skilled in the art to which the disclosure pertains.

### [Solution to Problem]

According to an embodiment, an electronic device may include a display, a processor, and a memory storing medication intake schedule information and instructions. The instructions may be configured to cause, when executed by the processor, the electronic device to: display a clock screen including an icon for at least one medication, based on the medication intake schedule information; identify a notification time for taking the at least one medication, based on the medication intake schedule information; identify an adjustment condition for a medication intake-related notification signal, based on the identified notification time; adjust an output timing of the notification signal for taking the at least one medication, based on the identified adjustment condition for the medication intake-related notification signal; and display a visual effect corresponding to the icon included in the clock screen, based on the adjusted output timing.

According to an embodiment, a method for outputting a notification signal for medication intake in an electronic device may include: displaying a clock screen including an icon for at least one medication, based on medication intake schedule information; identifying a notification time for taking the at least one medication, based on the medication intake schedule information; identifying an adjustment condition for a medication intake-related notification signal, based on the identified notification time; adjusting an output timing of the notification signal for taking the at least one medication, based on the identified adjustment condition for the medication intake-related notification signal; and displaying a visual effect corresponding to the icon included in the clock screen, based on the adjusted output timing.

According to an embodiment, a non-transitory computer-readable storage medium (or a computer program product) that stores one or more programs may be described. According to an embodiment, the one or more programs may include instructions that, when executed by a processor of an electronic device, perform operations of: displaying a clock screen including an icon for at least one medication based on medication intake schedule information; identifying a notification time for taking the at least one medication based on the medication intake schedule information; identifying an adjustment condition for a medication intake-related notification signal based on the identified notification time; adjusting an output timing of the notification signal for taking the at least one medication based on the identified adjustment condition for the medication intake-related notification signal; and displaying a visual effect corresponding to the icon included in the clock screen based on the adjusted output timing.

### [Advantageous Effects of Invention]

According to an embodiment, an electronic device may provide a notification (e.g., a notification signal) based on a medication intake management application (e.g., a program related to medication intake) in accordance with a set medication intake time, so that a user can correctly take a designated medication. For example, when another schedule is identified at the set medication intake time, the electronic device may adjust an output timing of the notification signal such that the notification signal for medication intake is output after the identified other schedule ends. The electronic device may adjust the output timing of the notification signal based on adjustment conditions for the medication intake-related notification signal to ensure correct intake of the designated medication. The notification signal may include at least one of a visual effect through a screen, an auditory effect through an audio signal, and/or a tactile effect through a vibration signal.

According to an embodiment, following the output of the notification signal, the electronic device may detect a movement of the electronic device, determine whether the medication intake is completed based on the detected movement, and automatically dismiss the medication intake-related notification when the medication intake is confirmed. By determining whether medication has been taken based on the detected movement, the electronic device can conveniently manage a schedule related to medication intake. As such, in managing the medication-related schedule, user convenience can be enhanced.

The effects that can be obtained from the disclosure are not limited to those mentioned above, and other effects not mentioned will be clearly understood from the following description by a person skilled in the art to which the disclosure pertains.

### [Brief Description of Drawings]

With regard to the description of the drawings, the same or similar reference numerals may be used for the same or similar components.
FIG. 1 is a block diagram of an electronic device in a network environment according to an embodiment of the disclosure.
FIG. 2 is a perspective view of a front surface of an electronic device according to an embodiment of the disclosure.
FIG. 3 is a perspective view of a rear surface of an electronic device according to an embodiment of the disclosure.
FIG. 4 is an exploded perspective view of an electronic device according to an embodiment of the disclosure.
FIG. 5 is an exemplary view illustrating a notification screen in which a visual effect is reflected in a user interface related to medication intake, according to an embodiment of the disclosure.
FIG. 6 is a block diagram of an electronic device according to an embodiment of the disclosure.
FIG. 7 is a flowchart illustrating a method of adjusting an output timing of a notification signal based on adjustment conditions for a medication intake-related notification signal and outputting a notification signal in which a visual effect is reflected, according to an embodiment of the disclosure.
FIG. 8 is an exemplary view illustrating a user interface including icons for medication to be taken based on a medication intake schedule, according to an embodiment of the disclosure.
FIG. 9A is a view illustrating an example of displaying a medication icon indicating that medication intake is completed in response to a user input, according to an embodiment of the disclosure.
FIG. 9B is a view illustrating an example of displaying a medication icon indicating that medication intake is skipped in response to a user input, according to an embodiment of the disclosure.
FIG. 9C is a view illustrating an example of displaying a medication icon indicating important medication intake in response to a user input, according to an embodiment of the disclosure.
FIG. 9D is a view illustrating an example in which a user interface changes over time, according to an embodiment of the disclosure.
FIG. 9E is a view illustrating an example of displaying a message indicating a state in which medication intake is completed, according to an embodiment of the disclosure.
FIG. 9F is a view illustrating an example of displaying a medication icon for medication to be taken in a state where a designated medication intake time has elapsed, according to an embodiment of the disclosure.
FIG. 10 is a view illustrating an example of adjusting an output timing of a notification signal based on adjustment conditions for a medication intake-related notification signal and outputting a notification signal in which a visual effect is reflected, according to an embodiment of the disclosure.
FIG. 11 is a view illustrating an example of outputting a medication intake-related notification signal via another electronic device linked with the electronic device in a state where the electronic device is not worn, according to an embodiment of the disclosure.
FIG. 12 is a view illustrating an example of displaying a notification message based on medication-related information and displaying a user interface in which a visual effect associated with the medication-related information is reflected, according to an embodiment of the disclosure.
FIG. 13 is a view illustrating an example of displaying a notification message based on medication-related information and displaying medical information related to medication intake, according to an embodiment of the disclosure.
FIG. 14 is a view illustrating an example of detecting a movement of an electronic device caused by medication intake and recognizing completion of medication intake based on the detected movement, according to an embodiment of the disclosure.
FIG. 15 is a view illustrating an example of identifying a user's body temperature and adjusting an output timing of a notification signal based on the identified body temperature, according to an embodiment of the disclosure.
FIG. 16 is a view illustrating an example of registering a medication by searching for a medication name and registering a medication by scanning a medication listed on a prescription with a camera, according to an embodiment of the disclosure.

### [Mode for the Invention]

Hereinafter, an embodiment of the disclosure will be described in detail with reference to the accompanying drawings so that the disclosure may be easily implemented by those skilled in the art to which the disclosure belongs. However, the disclosure may be implemented in various different forms and is not limited to embodiments described herein. In describing the drawings, the same or similar reference numerals may be used for the same or similar components. In addition, in the drawings and related descriptions, descriptions of well-known functions and configurations may be omitted for clarity and brevity.

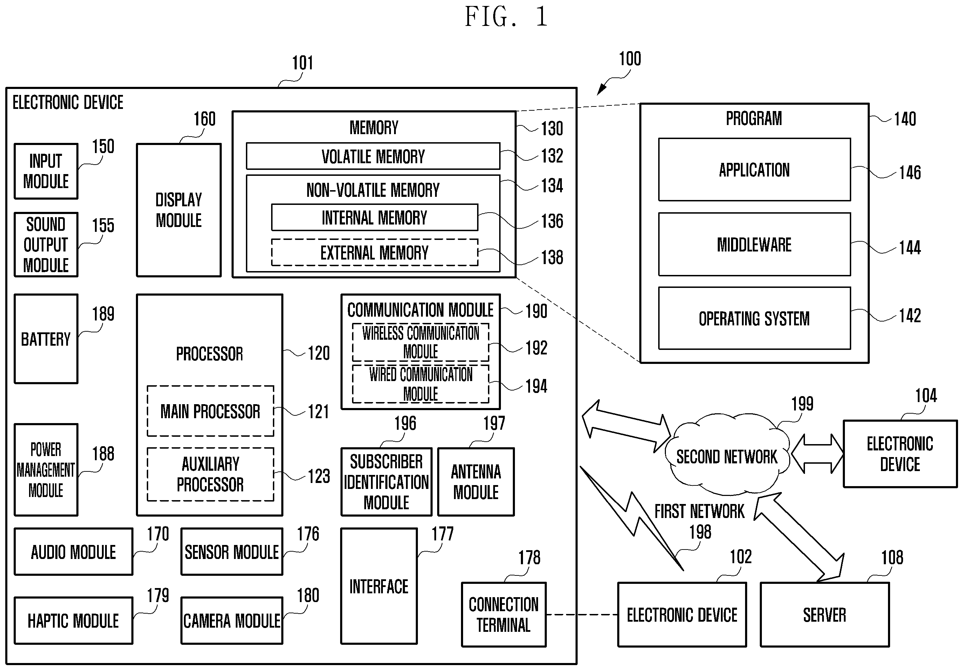

Fig. 1 is a block diagram illustrating an example electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102)(e.g., a speaker or a headphone) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultrareliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). For example, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band. For example, the plurality of antennas may include a patch array antenna and/or a dipole array antenna.

At least some of the above-described components may be coupled mutually and communicate signals (e.g., commands or data) therebetween via an interperipheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

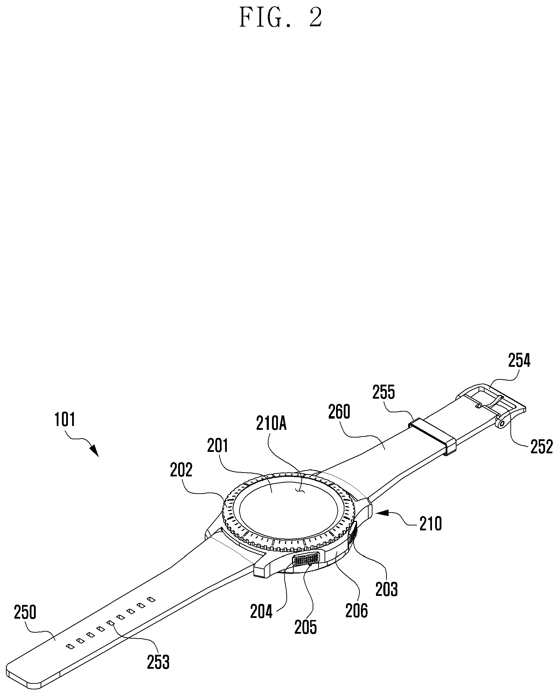

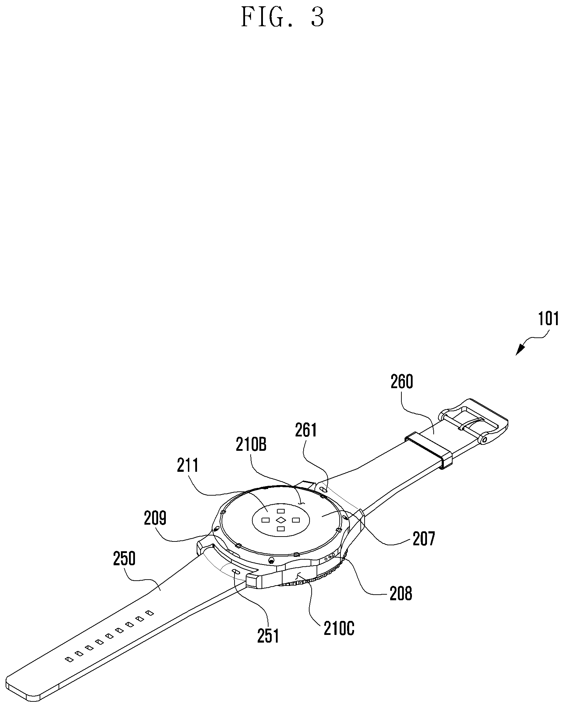

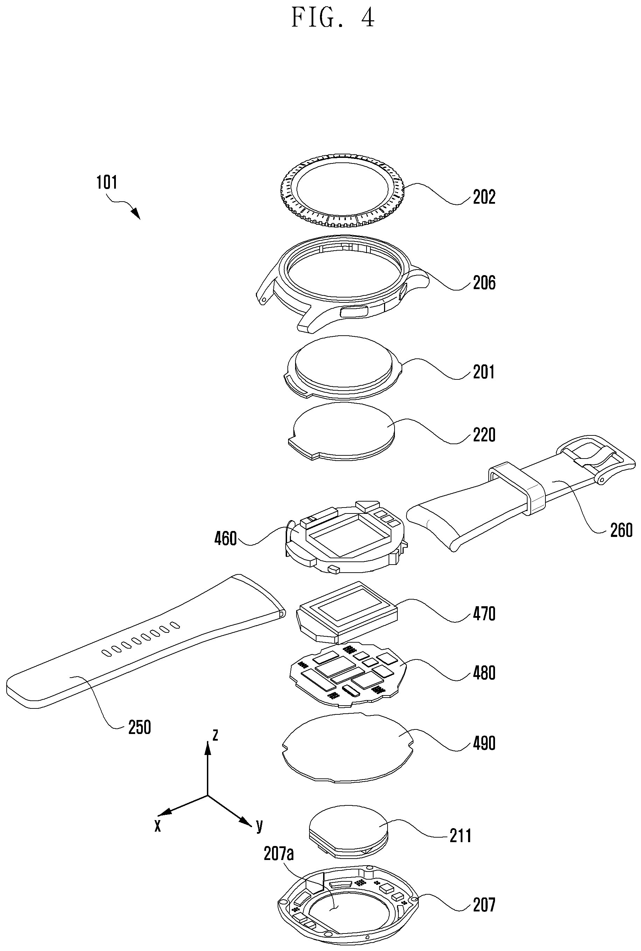

FIG. 2 is a front perspective view of an electronic device according to an embodiment of the disclosure. FIG. 3 is a rear perspective view of an electronic device according to an embodiment of the disclosure. FIG. 4 is an exploded perspective view of an electronic device according to an embodiment of the disclosure.

The electronic device 101 in FIG. 2 to FIG. 4 (for example, the electronic device 101 in FIG. 1) may be at least partially similar to the electronic device 101 in FIG. 1, or may further include other embodiments of the electronic device. For example, the electronic device 101 may include a wearable electronic device which can be worn on a part of a human body.

Referring to FIG. 2 and FIG. 3, the electronic device 101 according to an embodiment (for example, a wearable electronic device or a wearable watch) may include a housing 210 including a first surface (or front surface) 210A, a second surface (or rear surface) 210B, and a lateral surface 210C surrounding the space between the first surface 210A and the second surface 210B, and clamping members 250 and 260 connected to at least a part of the housing 210 and configured such that the electronic device 101 can be detachably clamped to a part of the user's body (for example, wrist or ankle). The clamping members 250 and 260 may be straps, for example, wound around the user's wrist such that the electronic device 101 is retained. In another embodiment (not illustrated), the housing may refer to a structure forming at least some of the first surface 210A, the second surface 210B, and the lateral sur5face 210C of the electronic device 101. According to an embodiment, at least a part of the first surface 210A may be formed by a substantially transparent front plate 201 (for example, a glass plate including various coating layers, or a polymer plate). The second surface 210B may be formed by a rear plate 207. The rear plate 207 may be formed by, for example, coated or colored glass, ceramic, polymer, metal (for example, aluminum, stainless steel (STS), or magnesium), or a combination of at least two of the above materials. The lateral surface 210C may be coupled to the front plate 201 and the rear plate 207, and may be formed by a lateral bezel structure (or (lateral member") 206 including a metal and/or a polymer. In some embodiments, the rear plate 207 and the lateral bezel structure 206 may be formed integrally, and may include the same material (for example, a metal material such as aluminum). The clamping members 250 and 260 may be made of various materials in various shapes. For example, integrated and multiple unit links may be formed to be movable with regard to each other by using, for example, a woven material, leather, rubber, synthetic resin, metal, ceramic, or a combination of at least two of the above materials.

According to an embodiment, the electronic device 101 may include at least one of a display (for example, the display module 160 in FIG. 1 and/or the display 220 in FIG. 4), audio modules 205 and 208 (for example, the audio module 170 in FIG. 1), a sensor module 211 (for example, the sensor module 176 in FIG. 1), key input devices 202, 203, and 204 (for example, the input module 150 in FIG. 1), and a connector hole 209 (for example, the connection terminal 176 in FIG. 1). In some embodiments, at least one of the components (for example, the key input devices 202, 203, and 204, the connector hole 209, or the sensor module 211) of the electronic device 101 may be omitted, or the electronic device 101 may additionally include other components. According to an embodiment, the components are not limited to the parts illustrated in FIG. 2 to FIG. 4.

The display 220 may be visually exposed, for example, through a corresponding portion of the front plate 201. For example, the user may identify at least one content displayed on the display 220 through the front plate 201. The display 220 may have a shape corresponding to the shape of the front plate 201, which may be one of a circle, an ellipse, and/or a polygon. The display 220 may be at least partially coupled to or disposed adjacent to a touch sensing circuit, a pressure sensor capable of measuring the intensity (pressure) of a touch, and or a fingerprint sensor.

The audio modules 205 and 208 may include a microphone hole 205 and a speaker hole 208. The microphone hole 205 may have a microphone disposed therein so as to acquire external sounds. In some embodiments, multiple microphones may be disposed such that the direction of sounds can be sensed. The speaker hole 208 may be used as an external speaker and a telephone speech receiver. In some embodiments, the speaker hole 208 and the microphone hole 205 may be implemented as a single hole, or a speaker (for example, a piezoelectric speaker) may be included without the speaker hole 208.

The sensor module 211 may generate an electric signal or a data value corresponding to the internal operating state of the electronic device 101, or external environment states. The sensor module 211 may include a biometric sensor module (for example, a biometric sensor, a heart rate monitor (HRM) sensor, an oxygen saturation sensor, and/or a blood glucose sensor) disposed toward the second surface 210B of the housing 210, for example. When the electronic device 101 is worn on a part of a human body (for example, on a wrist), the sensor module 211 may be disposed so as to at least partially contact the human body. The electronic device 101 may further include a sensor module not illustrated, for example, at least one of a gesture sensor, a gyro sensor, a barometric pressure sensor, a magnetic sensor, an acceleration sensor, a grip sensor, a color sensor, an infrared (IR) sensor, a biometric sensor, a temperature sensor, a humidity sensor, or an illuminance sensor.

The key input devices 202, 203, and 204 may include a wheel key 202 disposed so as to correspond to the first surface 210A of the housing 210 and to be able to rotate along at least one direction (for example, clockwise, counterclockwise), and/or side key buttons 203 and 204 disposed on the lateral surface 210C of the housing 210. The wheel key 202 may have a shape corresponding to the shape of the front plate 201. According to another embodiment, the electronic device 101 may not include some or all of the above-mentioned key input devices 202, 203, and 204, and the key input devices 202, 203, and 204 not included may be implemented on the display 220 as soft keys or touch keys.

The connector hole 209 may contain a connector (for example, a USB connector) for transmitting/receiving power and/or data with an external electronic device (for example, the electronic devices 102 and 104 in FIG. 1). The electronic device 101 may further include, for example, a connector cover (not illustrated) configured to cover at least a part of the connector hole 209 and to prevent external foreign materials from infiltrating the connector hole 209.

The clamping members 250 and 260 may be detachably clamped in at least a partial region of the housing 210 by using locking members 251 and 261. The clamping members 250 and 260 may include at least one of a retaining member 252, a retaining member fastening hole 253, a band guide member 254, and a band retaining ring 255. According to an embodiment, the electronic device 101 may remain clamped at least partially to a part of a human body (for example, on a wrist) by using the clamping members 250 and 260.

The retaining member 252 may be at least partially coupled to the retaining member fastening hole 253 such that the housing 210 and the clamping members 250 and 260 are retained on a part of the user's body (for example, on a wrist or an ankle). The band guide member 254 may be configured such that, when the retaining member 252 is fastened to the retaining member fastening hole 253, the range of movement of the retaining member 252 is limited, thereby guaranteeing that the electronic device 101 is clamped to a part of the human body while the clamping members 250 and 260 maintain close contact with a part of the user's body. The band retaining ring 255 may limit the range of movement of the clamping members 250 and 260 while the retaining member 252 and the retaining member clamping hole 253 remain fastened to each other.

Referring to FIG. 4, the electronic device 101 may include a lateral bezel structure 206, a wheel key 202, a front plate 201, a display 220, a retaining member 460 (for example, a support member), a battery 470, a printed circuit board 480, a sealing member 490, and clamping members 250 and 260. For example, the retaining member 460 may be disposed inside the electronic device 101 and at least partially coupled to the lateral bezel structure 206, or may be formed integrally with the lateral bezel structure 206. The retaining member 460 may be made of a metal material and/or a nonmetal (for example, polymer) material. The display 220 may be coupled to one surface of the retaining member 460, and the printed circuit board 480 may be coupled to the other surface thereof. According to an embodiment, the battery 470 may be disposed between the retaining member 460 and the printed circuit board 480, and the retaining member 460 and the printed circuit board 480 may be electrically connected to each other. The retaining member 460 and the printed circuit board 480 may be electrically connected by using a conductive member (for example, a screw or a metallic material). According to an embodiment, a processor (for example, the processor 120 in FIG. 1), a memory (for example, the memory 130 in FIG. 1), and/or an interface (for example, the interface 177 in FIG. 1) may be mounted on the printed circuit board 480. The processor 102 may include at least one of a central processing unit (CPU), an application processor (AP), a graphic processing unit (GPU), a sensor processor, or a communication processor (CP), for example.

The memory 130 may include, for example, a volatile memory or a nonvolatile memory. The interface may include, for example, at least one of a high definition multimedia interface (HDMI), a universal serial bus (USB) interface, an SD card interface, and/or an audio interface. The interface may connect, for example, the electronic device 101 and an external electronic device electrically or physically, and may include an USB connector, an SD card/multimedia card (MMC) connector, or an audio connector.

The battery 470 (for example, the battery 189 in FIG. 1) may be a device for supplying power to at least one component of the electronic device 101, and may include, for example, at least one of a primary battery which is not rechargeable, a secondary battery which is rechargeable, or a fuel cell. At least a part of the battery 470 may be disposed on substantially the same plane with the printed circuit board 480, for example. The battery 470 may be disposed integrally in the electronic device 101 or disposed to be attachable to/detachable from the electronic device 101.

The printed circuit board 480 may have at least one antenna (for example, an antenna module or a chip antenna) mounted thereon. For example, the at least one antenna may include at least one of a near field communication (NFC) antenna, a wirelessly charging antenna, and/or a magnetic secure transmission (MST) antenna. The at least one antenna may perform short-range communication with an external device, may wirelessly transmit/receive power necessary for charging, and may emit magnetically based signals including short-range communication signals or payment data. According to an embodiment, an antenna structure may be formed by at least some of the lateral bezel structure 206, the front plate 201, and/or the retaining member 460 or a combination thereof. According to an embodiment, the retaining member 460 and the printed circuit board 480 may be electrically connected, and an electronic field (E-field) may be formed based on the retaining member 460 and the printed circuit board 480. The electronic device 101 may transmit/receive communication signals, based on the formed E-field, and may use at least a part of the retaining member 460 as an antenna.

The sealing member 490 may be positioned between the lateral bezel structure 206 and the rear plate 207. The sealing member 490 may be configured to prevent external moisture and foreign materials from infiltrating the space surrounded by the lateral bezel structure 206 and the rear plate 207.

The sensor module 211 (for example, a biometric sensor) may be disposed adjacent to the rear plate 207. For example, when the electronic device 101 is worn on a part of the wearer's body (for example, on a wrist), the rear plate 207 may be disposed so as to at least partially contact at least a part of the wearer's body. The sensor module 211 may be disposed so as to face a part of the wearer's body (for example, wrist or skin). The sensor module 211 may emit signals in various wavelength band with regard to a part of the wearer's body. The electronic device 101 may acquire various pieces of biometric information (for example, information related to the heart rate, blood glucose-related information, and/or oxygen saturation), based on signals in the wavelength bands which are reflected, scattered, and/or absorbed by a part of the wearer's body. According to an embodiment, the rear plate 207 may have a rear window 207a coupled thereto at least partially such that the above-mentioned signals are transmitted.

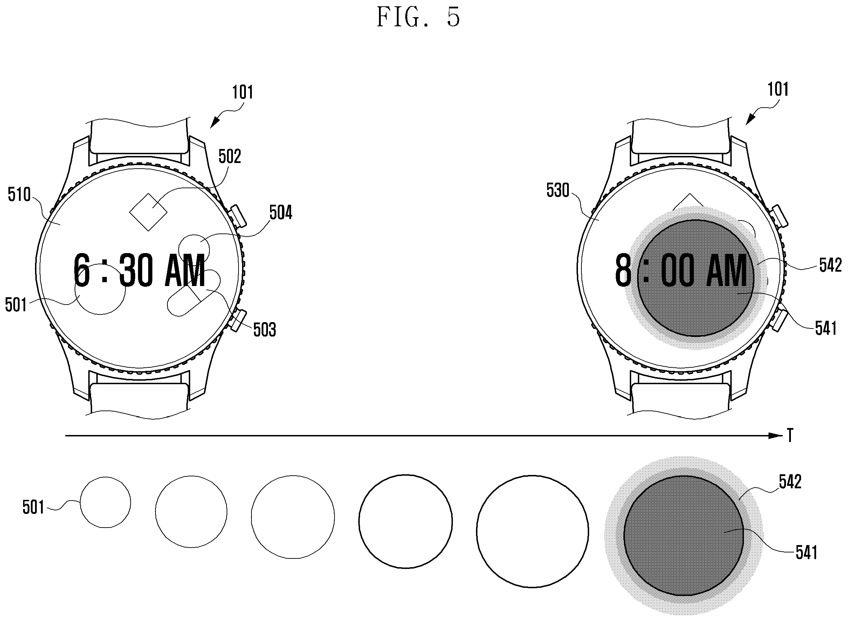

FIG. 5 is an exemplary view illustrating a notification screen in which a visual effect is reflected in a user interface related to medication intake, according to an embodiment of the disclosure.

The electronic device 101 (e.g., a wearable electronic device or a wearable watch) in FIG. 5 may be at least partially similar to the electronic device 101 in FIGS. 1 to 4, or may further include other embodiments of the electronic device 101. The operations illustrated in FIG. 5 may be understood as being performed by the electronic device 101.

Referring to FIG. 5, the electronic device 101 may include a wearable electronic device (e.g., a wearable watch) wearable on a user's wrist, and may display, through a display (e.g., the display module 160 in FIG. 1 or the display 220 in FIG. 2), a first user interface (UI) 510 including at least one medication icon 501, 502, 503, and 504 corresponding to medication to be taken, based on a medication intake schedule stored in a memory (e.g., the memory 130 in FIG. 1).

According to an embodiment, a processor (e.g., the processor 120 in FIG. 1) of the electronic device 101 may execute a medication intake management application (e.g., a program related to medication intake (medication to be taken) or a program for managing intake timing and dosage of medication to be taken) installed in the memory 130. The electronic device 101 may display, based on the medication intake management application, the first user interface 510 visually displaying at least one medication scheduled to be taken. Referring to FIG. 5, the electronic device 101 may identify four medications scheduled to be taken today, and may display the first user interface 510 including medication icons 501, 502, 503, and 504 corresponding to the identified medications. The electronic device 101 may obtain image information related to medication from a communicatively connected external electronic device (e.g., a server device (e.g., the server 108 in FIG. 1)), and based on the obtained image information, may display the medication icons 501, 502, 503, and 504 corresponding to the four medications scheduled to be taken. For example, the external electronic device may manage various types of information related to medication (e.g., ingredients, intake methods, effects, intake frequency, precautions, correlation with other medications, side effects, images, and/or shapes of the medication) by organizing them into a database (DB). According to an embodiment, the electronic device 101 may store the information related to medication obtained from the external electronic device in the memory 130 to manage it independently. For example, based on a main user using the electronic device 101, the electronic device 101 may independently manage at least one medication related to the main user.

According to an embodiment, the electronic device 101 may set a medication intake (e.g., medication to be taken) schedule for at least one medication 501, 502, 503, and 504 based on the medication intake management application, and may set at least one notification based on the set medication intake schedule. The electronic device 101 may implement at least one widget interface based on the medication intake management application, and may display the widget interface in a form that at least partially overlaps with a clock screen. For example, the electronic device 101 may include a wearable watch and display a clock screen showing the current time. For example, the electronic device 101 may display an analog-type clock screen or a digital-type clock screen through the display. The electronic device 101 may be configured such that at least one of the clock screen or the widget interface appears even in an always-on display (AOD) setting state. Referring to FIG. 5, in a state where the clock screen is being displayed, the electronic device 101 may display the widget interface in a form that at least partially overlaps with the clock screen. For example, the widget interface may include the at least one medication 501, 502, 503, and 504 for which the medication intake schedule has been set to be taken today.

According to an embodiment, the electronic device 101 may identify a medication intake-related notification corresponding to the at least one medication 501, 502, 503, and 504 included in the clock screen 510. For example, the electronic device 101 may identify a medication intake schedule of a first medication 501 included in the clock screen 510, and may identify that a first medication intake notification for the first medication 501 is set at 8:00 AM. As the output timing of the first medication intake notification approaches, the electronic device 101 may apply a visual effect related to the first medication 501. For example, as the output timing of the first medication intake notification draws closer, the size of the first medication 501 may gradually increase according to a predetermined ratio, and a visual emphasis effect 542 (e.g., a highlight effect, a color change, a texture change, a flickering effect, or an image change) may be applied and displayed. For example, the electronic device 101 may display the first medication 501 of a first size approximately one hour before the first medication intake notification, and may increase the size of the first medication 501 according to a predetermined ratio (e.g., approximately 10%) at every set time interval (e.g., approximately 10 minutes). Referring to FIG. 5, the electronic device 101 may display the first medication 541 of a second size at the timing of the first medication intake notification, and may apply the highlight effect 542 based on the first medication 541 of the second size.

According to an embodiment, the electronic device 101 may set a notification for at least one medication based on the medication intake management application, and may display the at least one medication 501, 502, 503, and 504 scheduled to be taken today based on the set notification. The electronic device 101 may display an interface including the at least one medication 501, 502, 503, and 504 in a form that at least partially overlaps with the clock screen. According to an embodiment, when a set notification time approaches, the electronic device 101 may apply and display a visual effect based on the corresponding medication such that the size of the corresponding medication gradually increases. For example, when the set notification time is reached, the electronic device 101 may apply and display the highlight effect 542 based on the medication corresponding to the notification time. According to an embodiment, the electronic device 101 may provide a method for a user to conveniently manage a schedule related to medication intake based on the medication intake management application.

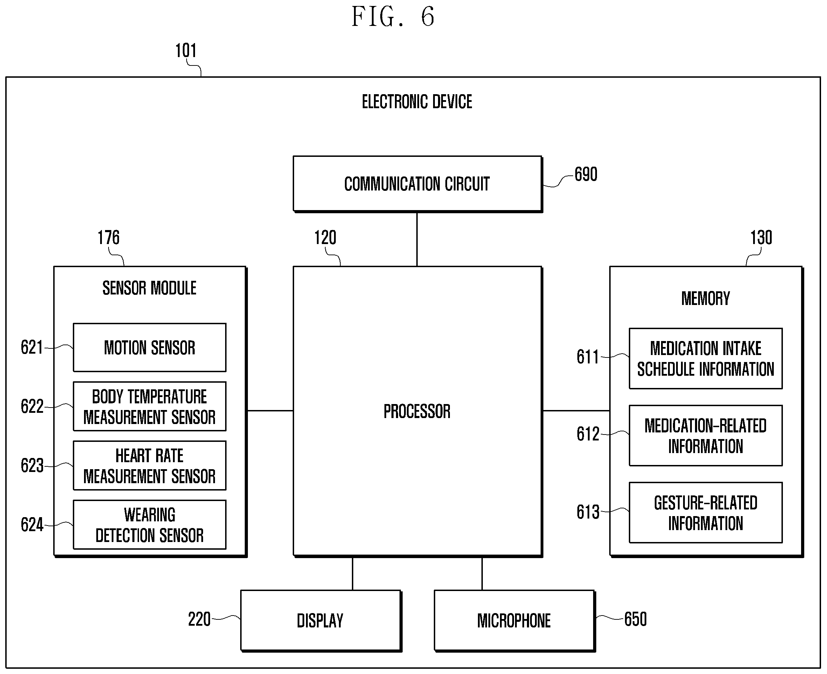

FIG. 6 is a block diagram of an electronic device according to an embodiment of the disclosure.

The electronic device 101 in FIG. 6 (e.g., the electronic device 101 in FIG. 1) may be at least partially similar to the electronic device 101 in FIG. 1, or may further include other embodiments of an electronic device. For example, the electronic device 101 may include a wearable electronic device wearable on a part of a human body. Like the electronic device 101 in FIG. 5, the electronic device 101 in FIG. 6 may include a wearable watch wearable on a part (e.g., a wrist) of a human body.

Referring to FIG. 6, the electronic device 101 may include a processor (e.g., the processor 120 in FIG. 1), a memory (e.g., the memory 130 in FIG. 1), a sensor module (e.g., the sensor module 176 in FIG. 1), a display 220 (e.g., the display module 160 in FIG. 1), a microphone 650 (e.g., the input module 150 in FIG. 1), and/or a communication circuit 690 (e.g., the communication module 190 in FIG. 1). For example, the sensor module 176 may include at least one of a motion sensor 621 for detecting a movement of the electronic device 101, a body temperature measurement sensor 622 for measuring a user's body temperature, a heart rate measurement sensor 623 for measuring a user's heart rate, and/or a wearing detection sensor 624 for determining whether the electronic device 101 is being worn. For example, the memory 130 may store at least one of medication intake schedule information 611, medication-related information 612, and/or gesture-related information 613.

According to an embodiment, the processor 120 of the electronic device 101 may execute a program (e.g., the program 140 in FIG. 1) stored in the memory 130 to control at least one other component (e.g., a hardware or software component) and perform various data processing or operations. According to an embodiment, the processor 120 may be operatively, functionally, and/or electrically connected to the memory 130, the sensor module 176, the microphone 650, the display 220, and/or the communication circuit 690.

According to an embodiment, the memory 130 may store a medication intake management application (e.g., a medication intake management program, or a program for managing intake timing (e.g., notification time) and dosage of medication to be taken) for managing medication taken by a user (e.g., medication intake). For example, the processor 120 may set a medication intake schedule for correctly taking at least one medication based on the medication intake management application.

According to an embodiment, the electronic device 101 may store, in the memory 130, the medication intake schedule information 611 in which a medication intake notification time is set, the medication-related information 612, and/or the gesture-related information 613 related to a movement of the electronic device 101. For example, the medication intake schedule information 611 may include at least one of a notification time for medication to be taken by a user (e.g., medication intake), a type of medication to be taken, the number of medications to be taken, the number of times the medication is taken, and/or information on whether the medication is a general medication or a prescription medication. The processor 120 may generate a medication intake notification signal based on the medication intake schedule information 611 and may output the medication intake notification signal in accordance with a set medication intake notification time. For example, the medication-related information 612 may include various types of information related to medication (e.g., ingredients, intake methods, effects, intake frequency, precautions, correlation with other medications, side effects, images, and/or shapes of the medication). The medication-related information 612 may be managed in the form of a database (DB) in an external electronic device (e.g., a server), and the electronic device 101 may obtain the medication-related information 612 from the external electronic device. The electronic device 101 may store the medication-related information 612 obtained from the external electronic device in the memory 130. The electronic device 101 may store, in the memory 130, information related to medication that the user takes frequently, periodically, or aperiodically. For example, the gesture-related information 613 may include movement information of the electronic device 101 caused by a movement of a wrist in a state where the electronic device 101 is worn on the user's wrist. For example, the gesture-related information 613 may include a gesture of the user lifting a cup to drink water from the cup and a gesture of putting the cup down on a table. The electronic device 101 may recognize a gesture for the electronic device 101 using the motion sensor 621 included in the sensor module 176, and may compare and analyze the recognized gesture with the gesture-related information 613 stored in the memory 130 to determine whether the user has drunk water for taking medication. When a movement related to medication intake is recognized, the electronic device 101 may provide a medication intake-related notification message to the user, and may determine whether medication intake is completed in response to a user input thereto.

According to an embodiment, the sensor module 176 may include at least one of the motion sensor 621 for detecting a movement of the electronic device 101, the body temperature measurement sensor 622 for measuring a user's body temperature, the heart rate measurement sensor 623 for measuring a user's heart rate, and/or the wearing detection sensor 624 for determining whether the electronic device 101 is worn on a part (e.g., a wrist) of the user's body. The motion sensor 621 may include various types of sensors for detecting a movement of the electronic device 101. For example, the motion sensor 621 may include at least one of a gesture sensor, a gyro sensor, a 6-axis sensor, an acceleration sensor, and/or a proximity sensor. The motion sensor 621 may detect a movement of the electronic device 101 worn on the user's wrist. In particular, the motion sensor 621 may distinguishably detect a movement of the user lifting a cup and a movement of drinking water from the cup. According to an embodiment, the electronic device 101 may recognize a medication intake-related gesture of the user based on the motion sensor 621, and may determine that medication has been correctly taken in response to the recognition of the medication intake-related gesture. The body temperature measurement sensor 622 may include a temperature sensor for measuring a user's body temperature. For example, the electronic device 101 may be worn in a form of being in physical contact with a human body (e.g., a wrist), and the body temperature measurement sensor 622 may be disposed in a form of being at least partially in contact with the human body. The processor 120 may measure and identify the user's body temperature periodically or aperiodically using the body temperature measurement sensor 622. The heart rate measurement sensor 623 may include an electrocardiogram (ECG) sensor that detects an electrical signal according to a heartbeat and/or a photoplethysmography (PPG) sensor (e.g., an optical blood flow measurement sensor, an optical volume pulse wave measurement sensor, or an optical volume measurement sensor) that measures blood flow using light. For example, the electronic device 101 may measure and identify the user's heart rate periodically or aperiodically using the heart rate measurement sensor 623. For example, when the user has a meal (e.g., ingesting or eating food), the heart rate may fluctuate, and the processor 120 may determine whether the user has ingested food based on the fluctuated heart rate. The wearing detection sensor 624 may determine whether the electronic device 101 is in a state of being worn on a part (e.g., a wrist) of the user's body. For example, the electronic device 101 may use the wearing detection sensor 624 to distinguishably recognize whether the electronic device 101 is in a state of being correctly worn on the user's wrist or in a state of being detached from the user's wrist.

According to an embodiment, the display 220 may display a user interface (UI) implemented based on the medication intake management application. For example, the user interface may be implemented based on a shape (e.g., a clock screen) and size of the display 220, and may include a medication icon corresponding to medication to be taken by the user.

According to an embodiment, the microphone 650 may be a component for obtaining an external audio signal. For example, the processor 120 may at least partially activate the microphone 650, and may obtain an external audio signal (e.g., a user's voice signal, noise generated in an external environment, or a sound of pouring water into a cup) using the activated microphone 650. According to an embodiment, the electronic device 101 may at least partially activate the microphone 650 in response to the output of the medication intake notification signal, and may recognize a sound of pouring water into a cup using the microphone 650. Based on the audio signal obtained through the microphone 650, the electronic device 101 may infer and determine whether medication has been taken.

According to an embodiment, the communication circuit 690 may be a component that operatively connects the electronic device 101 and an external electronic device (e.g., the server 108 in FIG. 1). For example, the processor 120 of the electronic device 101 may be operatively connected for communication with the external electronic device through the communication circuit 690, and may obtain the medication-related information 612 from the external electronic device.

According to an embodiment, the electronic device 101 may implement a user interface (e.g., a widget interface) related to a user's medication intake, based on the medication intake schedule information 611 and the medication-related information 612, and may display the user interface through the display 220.

According to an embodiment, the processor 120 of the electronic device 101 may identify a notification (e.g., a medication intake schedule or medication intake notification information) for at least one medication set based on the medication intake management application, and may display at least one medication scheduled to be taken today based on the identified notification. The notification for the at least one medication may be stored in the medication intake schedule information 611 of the memory 130. The processor 120 may implement a user interface corresponding to a clock screen (e.g., a user interface including at least one medication scheduled for intake), based on the medication intake schedule information 611, and may display the user interface through the display 220.

According to an embodiment, when a notification time (e.g., an output time of a notification signal) approaches, the electronic device 101 may apply and display a visual effect based on a corresponding medication such that the size of the corresponding medication gradually increases. For example, when a set notification time is reached, the electronic device 101 may apply and display a highlight effect based on the corresponding medication. According to an embodiment, the electronic device 101 may provide a method for a user to conveniently manage a schedule related to medication intake, based on the medication intake management application.

According to an embodiment, the electronic device 101 may identify adjustment conditions for a medication intake-related notification signal for at least one medication. For example, the adjustment conditions for the medication intake-related notification signal may determine whether to adjust an output timing of the medication intake-related notification signal, based on at least one of biometric information indicating one of an exercise state and a sleeping state, location information of the electronic device 101, and/or other schedule information. For example, regarding a medication intake-related notification time, if there is another schedule (e.g., a meeting schedule, another appointment, or a travel schedule) set to overlap with the notification time, the processor 120 may adjust an output time (e.g., an output timing) for the medication intake-related notification signal. For example, the processor 120 may identify the medication intake schedule information 611 based on the medication intake management application, identify schedule information stored in a calendar application (e.g., another application or a schedule-related application), and identify a time when the medication intake schedule information 611 and the schedule information at least partially overlap. For example, if a medication intake-related notification time (e.g., an output timing of a notification signal) is included within a time range for which a meeting is scheduled, the processor 120 may adjust the output timing at which the medication intake-related notification time is output. The processor 120 may adjust the output timing to be delayed so that the medication intake-related notification signal is output after the time the meeting is ended. For example, the processor 120 may output the medication intake-related notification signal after the set meeting time has elapsed.

As another example, the processor 120 may identify a user's physical state (e.g., an exercise state and/or a sleeping state) at a medication intake-related notification time, and may adjust an output time (e.g., an output timing) of the medication intake notification time according to the identified physical state. For example, the processor 120 may identify, based on the medication intake notification time, at least one of a user's body temperature information measured using the body temperature measurement sensor 622 and/or the user's heart rate information measured using the heart rate measurement sensor 623, and may determine whether an adjustment condition for a medication intake-related notification signal for the user is satisfied. For example, if the user's heart rate exceeds a set heart rate at a timing when the medication intake notification signal is output, the processor 120 may determine that the adjustment condition for the medication intake-related notification signal is satisfied, and may postpone the output timing of the medication intake-related notification signal. A situation in which the user's heart rate exceeds the set heart rate may include a situation in which the user is exercising, a situation in which the user is excited, and/or a situation in which it is difficult for the user to correctly take medication. The processor 120 may periodically or aperiodically check the user's heart rate, and may output the postponed medication intake-related notification signal in a situation where the user's heart rate falls below the set heart rate. As another example, at a timing when the medication intake notification signal is output, the processor 120 may identify whether the user is in a sleeping state, based on the body temperature information and the heart rate information, and may determine that the adjustment condition for the medication intake-related notification signal is satisfied in response to identification of the sleeping state. The processor 120 may postpone the output timing of the medication intake-related notification signal. According to various embodiments, the electronic device 101 may adjust the output time of the medication intake notification signal in consideration of the user's physical state (e.g., an exercise state or a sleeping state).

According to an embodiment, in managing the medication intake schedule related to medication intake, the electronic device 101 may consider other schedules and a user's physical state (e.g., an exercise state and/or a sleeping state) together, and may adjust an output time (e.g., an output timing) of the medication intake-related notification signal according to adjustment conditions for the medication intake-related notification signal. The electronic device 101 may adjust the output time of the medication intake-related notification signal so that the user can conveniently manage the medication intake schedule.

According to an embodiment, the electronic device (e.g., the electronic device 101 in FIG. 1, a wearable electronic device, or a wearable watch) may include the display 220, the processor 120, and the memory 130 that stores the medication intake schedule information 611 and instructions. The instructions may be configured to cause, when executed by the processor 120, the electronic device 101 to: display a clock screen including an icon for at least one medication based on the medication intake schedule information 611; identify a notification time for taking the at least one medication based on the medication intake schedule information 611; identify an adjustment condition for a medication intake-related notification signal based on the identified notification time; adjust an output timing of the notification signal for taking the at least one medication based on the identified adjustment condition for the medication intake-related notification signal; and display a visual effect corresponding to the icon included in the clock screen based on the adjusted output timing.

According to an embodiment, the electronic device 101 may further include the motion sensor 621 and the microphone 650. The instructions may be configured to cause, when executed by the processor 120, the electronic device 101 to: execute a recording function for the microphone 650 in response to the output of the notification signal; determine whether the at least one medication has been taken, based on at least one of an audio signal obtained using the recording function or gesture-related information obtained based on the motion sensor 621; and dismiss a notification output function according to the notification time in response to confirming that the at least one medication has been taken.

According to an embodiment, the instructions may be configured to cause, when executed by the processor 120, the electronic device 101 to: display a medication intake-related notification message in response to confirming that the at least one medication has been taken based on at least one of the audio signal or the gesture-related information; and change a parameter value indicating whether the at least one medication has been taken, in response to a user input for the notification message.

According to an embodiment, the visual effect corresponding to the icon may include, for the icon, a change in size, a change in color, a change in shape, an emphasis effect (e.g., a highlight effect), and a display of precautions related to medication.

According to an embodiment, the adjustment condition for the medication intake-related notification signal may determine whether to adjust an output timing of the medication intake-related notification signal, based on at least one of adjusted biometric information for the notification signal (e.g., heart rate information, body temperature information, or electrocardiogram (ECG) information), location information of the electronic device 101 (e.g., a medication intake location according to a medication intake history), and/or other schedule information (e.g., during a meeting, during travel, or during a conversation).

According to an embodiment, the electronic device 101 may further include a body temperature measurement sensor 622 for measuring a user's body temperature and a heart rate measurement sensor 623 for measuring a user's heart rate. The instructions may be configured to cause, when executed by the processor 120, the electronic device 101 to: identify that the user is in an exercise state, based on at least one of body temperature information measured using the body temperature measurement sensor 622 and heart rate information measured using the heart rate measurement sensor 623; and adjust the output timing of the notification signal for taking the at least one medication in response to identifying the exercise state.

According to an embodiment, the instructions may be configured to cause, when executed by the processor 120, the electronic device 101 to: identify heart rate information by the heart rate measurement sensor 623 and movement information of the electronic device 101 by the motion sensor 621; identify that a user wearing the electronic device is in a sleeping state, based on the heart rate information and the movement information; and adjust the output timing of the notification signal for taking the at least one medication in response to identifying the sleeping state.

According to an embodiment, the electronic device 101 may further include a wearing detection sensor 624 and a communication circuit 690. The instructions may be configured to cause, when executed by the processor 120, the electronic device 101 to: determine whether the electronic device 101 is in a state of being worn on a part of the user's body, using the wearing detection sensor 624; identify other electronic device 102 or 104 operatively connected to the electronic device 101 through the communication circuit 690 in response to the electronic device 101 being in an unworn state; and output a notification signal related to taking the at least one medication, based on the identified other electronic device 102 or 104.

According to an embodiment, the instructions may be configured to cause, when executed by the processor 120, the electronic device 101 to: receive medication-related information from an external electronic device 108; and display precautions related to taking the at least one medication through the display 220, based on the received medication-related information.

According to an embodiment, the instructions may be configured to cause, when executed by the processor 120, the electronic device 101 to: when a user's activity is identified based on information (e.g., schedule information) received from other application (e.g., a calendar application or a schedule-related application), adjust the output timing of the notification signal for taking the at least one medication based on the information received from the other application.

According to an embodiment, the electronic device 101 may further include a camera 180. The instructions may be configured to cause, when executed by the processor 120, the electronic device 101 to: scan medication intake information related to the at least one medication, using the camera 180; and register the medication intake schedule information 611 corresponding to the at least one medication, based on the scanned medication intake information.

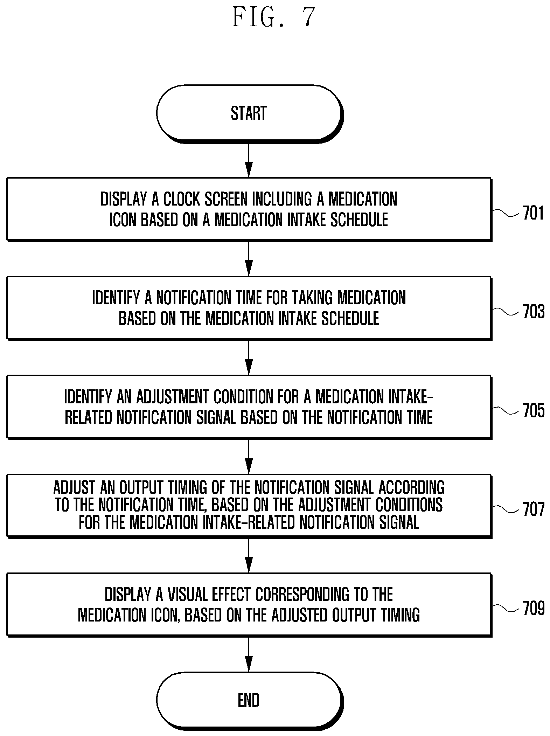

FIG. 7 is a flowchart illustrating a method of adjusting an output timing of a notification signal based on adjustment conditions for a medication intake-related notification signal and outputting a notification signal in which a visual effect is reflected, according to an embodiment of the disclosure.

In the following embodiment, respective operations may be performed sequentially, but are not necessarily performed sequentially. For example, the order of respective operations may be changed, or at least two operations may be performed in parallel.

According to an embodiment, operations 701 to 709 may be understood as being performed by a processor (e.g., the processor 120 in FIGS. 1 and 6) of an electronic device (e.g., the electronic device 101 in FIGS. 1 to 6). The electronic device 101 in FIG. 7 may be at least partially similar to the electronic device 101 in FIGS. 1 to 6, or may further include other embodiments of an electronic device.

According to an embodiment, the electronic device 101 may include a wearable electronic device wearable on a part (e.g., a wrist) of a user's body. For example, the electronic device 101 may include a wearable watch worn on the user's wrist. When worn, the electronic device 101 may maintain a state of being at least partially in contact with the user's skin. The electronic device 101 may display at least one medication icon related to medication intake through a display (e.g., the display 220 in FIG. 6), and when a set medication intake notification time is reached, the electronic device 101 may output a notification signal related to medication intake. The electronic device 101 may apply a visual effect to the medication icon. For example, the size of the medication icon may gradually increase as the medication intake notification time approaches, and when the medication intake notification time is reached, an emphasis effect may be reflected and displayed on the medication icon. According to an embodiment, the electronic device 101 may display a user interface related to medication so that the user can intuitively identify a schedule related to medication intake. The electronic device 101 may display the medication-related user interface so that the user can correctly take medication in accordance with the schedule related to medication intake.

According to an embodiment, in outputting the medication intake-related notification signal, the electronic device 101 may consider an adjustment condition for the medication intake-related notification signal (e.g., a condition for adjusting an output timing of the medication intake-related notification signal), and may adjust an output time of the medication intake-related notification signal based on the adjustment condition for the medication intake-related notification signal. For example, the adjustment condition for the medication intake-related notification signal may determine whether to adjust the output timing of the medication intake-related notification signal, based on at least one of biometric information (e.g., heart rate information, body temperature information, or ECG information) by which one of an exercise state and a sleeping state is identified, location information of the electronic device 101 (e.g., a medication intake location according to a medication intake history), and/or other schedule information (e.g., information obtained based on being in a meeting, being in travel, being in a conversation, or other applications). According to an embodiment, the electronic device 101 may provide a method for the user to conveniently manage the output timing of the notification signal related to medication intake (e.g., a schedule related to medication intake).

According to an embodiment, the operation of adjusting the output timing of the medication intake-related notification signal may be replaced with at least one of an updating operation, a postponing operation, a regulating operation, and/or a changing operation in relation to the output timing of the notification signal.

In operation 701, the processor 120 of the electronic device 101 may display a clock screen including a medication icon (e.g., the medication icons 501, 502, 503, and 504 in FIG. 5) based on a medication intake schedule (e.g., the medication intake schedule information 611 in FIG. 6). For example, the electronic device 101 includes a wearable watch and may display a clock screen showing the current time through the display 220. The processor 120 may identify at least one medication scheduled to be taken today and a medication intake notification time for taking the at least one medication, based on the medication intake schedule information 611. For example, the processor 120 may obtain the medication icons 501, 502, 503, and 504 (e.g., the medication-related information 612 in FIG. 6) corresponding to the identified at least one medication from an external electronic device (e.g., the server 108) that manages a medication-related database (DB), and may implement a user interface and/or a widget interface including the obtained medication icons 501, 502, 503, and 504. According to an embodiment, the electronic device 101 may display, through the display 220, a user interface corresponding to the clock screen (e.g., a user interface including the medication icons 501, 502, 503, and 504).

In operation 703, the processor 120 may identify a notification time for taking medication based on the medication intake schedule. For example, a notification time for medication intake may be individually set for each of the at least one medication icon 501, 502, 503, and 504. For example, a first notification time may be set for a first medication corresponding to the first medication icon 501, and a second notification time may be set for a second medication corresponding to the second medication icon 502.

In operation 705, the processor 120 may identify an adjustment condition for a medication intake-related notification signal based on the identified notification time. For example, the adjustment condition for the medication intake-related notification signal may determine whether to adjust an output timing of the medication intake-related notification signal, based on at least one of biometric information (e.g., heart rate information, body temperature information, or ECG information) by which one of an exercise state and a sleeping state is identified, location information of the electronic device 101 (e.g., a medication intake location according to a medication intake history), and/or other schedule information (e.g., information obtained based on being in a meeting, being in travel, being in a conversation, or other applications). According to an embodiment, the adjustment condition for the medication intake-related notification signal may include a condition for adjusting the output timing of the medication intake-related notification signal and a condition for determining whether a user's state is a state suitable for taking medication.

According to an embodiment, the condition for adjusting the output timing of the medication intake-related notification signal may include a situation in which another schedule is being performed at the output timing of the notification signal, or a situation in which the user is in another state (e.g., an exercise state and/or a sleeping state) at the output timing of the notification signal. According to an embodiment, the electronic device 101 may adjust the output timing of the notification signal when another schedule is identified, or when the user's exercise state and the user's sleeping state are identified, based on the output timing of the medication intake-related notification signal. The electronic device 101 may adjust the output timing of the notification signal so that the notification signal is output after the other schedule is ended, after the exercise state is ended, or after the sleeping state is ended.

For example, the medication intake management application may request other schedules of the user from a calendar application that manages the other schedules of the user, and may obtain other schedules registered by the user (e.g., schedules related to a meeting, a conference, or a personal activity). The processor 120 may determine whether the output timing of the medication intake-related notification signal is included, based on the other schedules of the user. For example, if it is identified that the output time of the medication intake-related notification signal is included in the user's meeting time, the user may currently be in a situation where taking medication is difficult. The electronic device 101 may adjust the output timing of the notification signal so that the medication intake-related notification signal is output after the meeting is ended.

For example, the medication intake management application may obtain movement information based on a motion sensor (e.g., the motion sensor 621 in FIG. 6) and heart rate information based on a heart rate measurement sensor (e.g., the heart rate measurement sensor 623 in FIG. 6), and may determine whether the user is exercising or whether the user is sleeping based on the obtained movement information and heart rate information. For example, if it is identified that the user is exercising (e.g., an exercise state) at the output timing of the medication intake-related notification signal, the user may currently be in a situation where taking medication in accordance with the notification time is difficult. The electronic device 101 may adjust the output timing of the notification signal so that the medication intake-related notification signal is output after the exercise state is ended. As another example, if it is identified that the user is sleeping (e.g., a sleeping state) at the output timing of the medication intake-related notification signal, the user may currently be in a situation where taking medication in accordance with the notification time is difficult. The electronic device 101 may adjust the output timing of the notification signal so that the medication intake-related notification signal is output after the sleeping state is ended (e.g., in a state where the user has woken up).

According to an embodiment, the condition for determining whether the user's state is a state suitable for taking medication may include a situation of determining whether the user is able to take medication at the current time and in the current situation.

For example, the medication intake management application may request the user's biometric information from an application related to biometric information, and may obtain the user's biometric information. The processor 120 may determine whether the user is currently in a state capable of taking medication (e.g., a state suitable for taking medication), based on the user's biometric information. For example, if it is identified that the user's heart rate exceeds a set heart rate, the user may currently be in a situation where taking medication is difficult. The processor 120 may display a guidance message related to medication intake in response to identifying the situation where taking medication is difficult. According to an embodiment, when it is identified that the user's heart rate falls to or below the set heart rate, the electronic device 101 may output a medication intake-related notification signal.

As another example, the medication intake management application may request location information for the electronic device 101 from an application related to location information of the electronic device 101, and may obtain location information related to a location where the user previously took medication. The processor 120 may determine whether the user is currently in a state capable of taking medication (e.g., a state suitable for taking medication), based on the medication intake-related location information. For example, if it is identified that the user has visited an unfamiliar place that the user has not visited before, the user may currently be in a situation where taking medication is difficult. The processor 120 may display a guidance message related to medication intake in response to identifying the situation where taking medication is difficult. According to an embodiment, when the location information is changed and it is identified that the location is a place where medication was taken in the past, the electronic device 101 may output the medication intake-related notification signal.

In operation 707, the processor 120 may adjust an output timing of the notification signal according to the notification time, based on the adjustment conditions for the medication intake-related notification signal. For example, if it is identified that the user is in a condition capable of taking medication (e.g., if the adjustment condition for the medication intake-related notification signal is satisfied), the electronic device 101 may output the medication intake-related notification signal in accordance with a set medication intake schedule. Conversely, if it is identified that the user is in a condition where taking medication is difficult (e.g., if the adjustment condition for the medication intake-related notification signal is not satisfied), the electronic device 101 may postpone the output timing of the medication intake-related notification signal. For example, if the medication intake-related notification time overlaps with a meeting time, the processor 120 may postpone the output timing of the medication intake-related notification signal to after the meeting is ended so that the medication intake-related notification signal is output after the meeting is ended. For example, if it is determined that taking medication is difficult based on the user's biometric information (e.g., heart rate information) measured at the medication intake-related notification time, the processor 120 may postpone the output timing of the medication intake-related notification signal to after the biometric information is recovered so that the medication-related notification signal is output after the user's biometric information is recovered within a normal range. As another example, if it is identified that the user is sleeping at the medication intake notification time (e.g., at the medication intake notification timing), the processor 120 may adjust the output timing of the medication intake-related notification signal so that the medication intake notification signal is output after the user has woken up.

In operation 709, the processor 120 may display a visual effect corresponding to the medication icon, based on the adjusted output timing. For example, the processor 120 may apply the visual effect to the medication icon such that the size of the medication icon gradually increases as the notification time (e.g., the output timing of the notification signal) approaches. When the notification time is reached, the processor 120 may apply and display an emphasis effect (e.g., a color change, a shape change, or a reflection of a highlight effect) to the medication icon.

According to an embodiment, the processor 120 of the electronic device 101 may implement a user interface (e.g., a clock screen) related to medication to be taken, based on the medication intake management application, and may display the user interface including at least one medication icon according to the medication intake schedule information 611. The processor 120 may adjust an output time (e.g., a medication intake time) of the medication intake notification signal for medication to be taken, based on the adjustment conditions for the medication intake-related notification signal. When the medication intake time is reached, the processor 120 may apply a visual effect based on the at least one medication icon.

According to an embodiment, the processor 120 may detect a movement of the electronic device 101 (e.g., a user's wrist movement), using a motion sensor (e.g., the motion sensor 621 in FIG. 6), and may determine whether at least one medication has been taken, based on the detected movement of the electronic device 101. For example, if the movement of the electronic device 101 matches a movement of taking medication within a set time after the medication intake notification signal is output, the processor 120 may determine that the user has taken the medication. When the medication intake is confirmed, the processor 120 may dismiss the medication intake notification signal for the medication.

According to an embodiment, the processor 120 may obtain an external audio signal using a microphone (e.g., the microphone 650 in FIG. 6), and may determine whether at least one medication has been taken, based on the obtained audio signal. For example, if a sound of pouring water into a cup and a sound of putting the cup down on a table after the user drinks water are identified within a set time after the medication intake notification signal is output, the processor 120 may determine that the user has taken the medication. When the medication intake is confirmed, the processor 120 may dismiss the medication intake notification signal for the medication.

According to an embodiment, in response to confirming that the at least one medication has been taken, the processor 120 may display a notification message related to medication intake through the display 220, and may change a parameter value indicating whether the at least one medication has been taken, in response to a user input for the notification message.

According to an embodiment, in the electronic device 101, a health-related application for managing biometric information related to the user and a medication intake schedule-related application for managing a medication intake schedule may be individually installed. For example, the medication intake management application (e.g., a medication intake management program) may include the health-related application and the medication intake schedule-related application. The processor 120 may identify the user's activity (e.g., state information, an exercise state, or a sleeping state) based on the health-related application, and may identify a notification time related to medication intake based on the medication intake schedule-related application. The processor 120 may adjust the output timing of the medication intake-related notification signal, based on the identified user's activity and the notification time related to medication intake.

According to an embodiment, the electronic device 101 may adjust the output timing of the notification signal related to medication to be taken, based on the adjustment conditions for the medication intake-related notification signal. The electronic device 101 may determine whether medication to be taken has been correctly taken, using various sensors, and when correct intake is confirmed, may dismiss the medication intake notification signal for the medication to be taken. The electronic device 101 may provide a method for conveniently managing a schedule related to medication intake. According to an embodiment, when the user manages the medication intake-related schedule, user convenience may be improved.

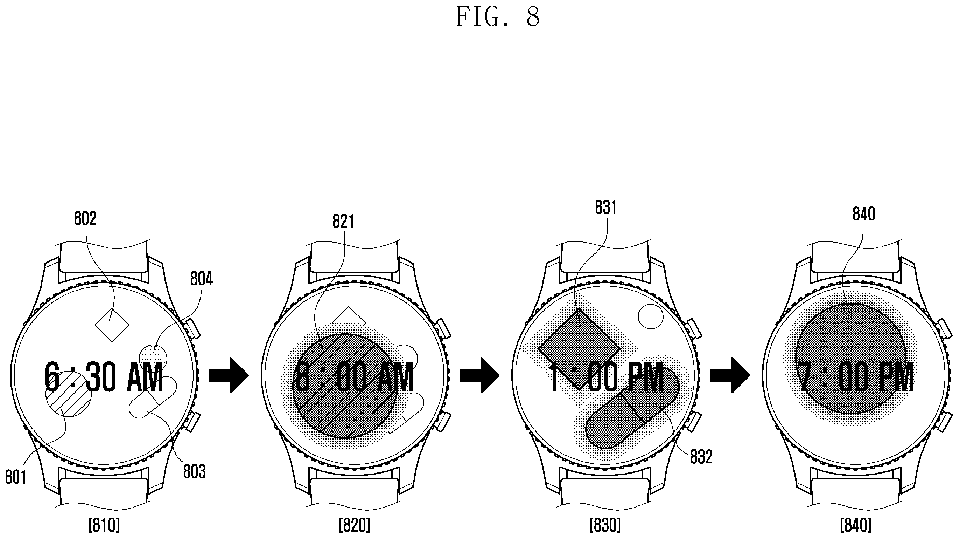

FIG. 8 is an exemplary view illustrating a user interface including icons for medication to be taken based on a medication intake schedule, according to an embodiment of the disclosure.

The electronic device 101 in FIG. 8 may be at least partially similar to the electronic device 101 in FIGS. 1 to 6, or may further include other embodiments of an electronic device.

According to an embodiment, the electronic device 101 may include a wearable electronic device wearable on a part (e.g., a wrist) of a user's body. For example, the electronic device 101 may include a wearable watch worn on the user's wrist. When worn, the electronic device 101 may maintain a state of being at least partially in contact with the user's skin. The electronic device 101 may display at least one medication icon related to medication intake through a display (e.g., the display 220 in FIG. 6), and when a set medication intake notification time is reached, the electronic device 101 may output a notification signal related to medication intake. The electronic device 101 may apply a visual effect to the medication icon.

Referring to FIG. 8, a first screen 810 may include at least one medication icon 801, 802, 803, and 804 corresponding to medications scheduled to be taken today by the user, and current time information (e.g., 6:30 AM). For example, a first medication corresponding to a first medication icon 801 is scheduled to be taken at a first time (e.g., 8:00 AM). A processor (e.g., the processor 120 in FIG. 6) of the electronic device 101 may set a first medication intake notification signal to be output in accordance with the first time. For example, a second medication corresponding to a second medication icon 802 and a third medication corresponding to a third medication icon 803 are scheduled to be taken at a second time (e.g., 2:00 PM). The processor 120 may set a second medication intake notification signal to be output in accordance with the second time. For example, a fourth medication corresponding to a fourth medication icon 804 is scheduled to be taken at a third time (e.g., 7:00 PM). The processor 120 may set a third medication intake notification signal to be output in accordance with the third time.

When the first time (e.g., 8:00 AM) is reached on a second screen 820, the processor 120 may adjust the first medication icon 801 into an adjusted first icon 821, to which a visual effect (e.g., an emphasis effect) is applied along with an increase in size, and display the same. For example, the first medication icon 801 may be displayed with an effect in which the size gradually increases as the first time approaches. According to an embodiment, the electronic device 101 may apply a visual effect to the first medication icon 801 scheduled for intake at the first time, and may display the first medication icon 801 as the adjusted first icon 821.

When the second time (e.g., 1:00 PM) is reached on a third screen 830, the processor 120 may adjust the second medication icon 802 and the third medication icon 803 as an adjusted second icon 831 and an adjusted third icon 832, respectively, to which a visual effect (e.g., an emphasis effect) is applied along with an increase in size, and display the same. For example, the second medication icon 802 and the third medication icon 803 may be displayed with an effect in which the sizes gradually increase as the second time approaches. According to an embodiment, the electronic device 101 may apply a visual effect to the second and third medication icons 802 and 803 scheduled for intake at the second time, and may display the second and third medication icons 802 and 803 as the adjusted second and third icons 831 and 832.

When the third time (e.g., 7:00 PM) is reached on a fourth screen 840, the processor 120 may adjust the fourth medication icon 804 as an adjusted fourth icon 841, to which a visual effect (e.g., an emphasis effect) is applied along with an increase in size, and display the same. For example, the fourth medication icon 804 may be displayed with an effect in which the size gradually increases as the fourth time approaches. According to an embodiment, the electronic device 101 may apply a visual effect to the fourth medication icon 804 scheduled for intake at the fourth time, and may display the fourth medication icon 804 as the adjusted fourth icon 841.

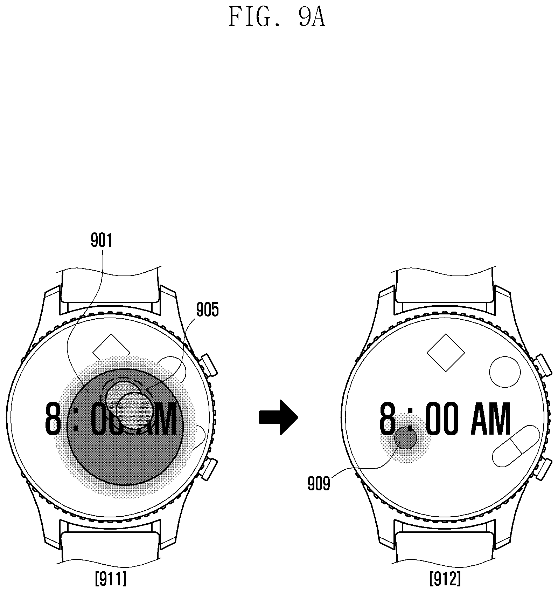

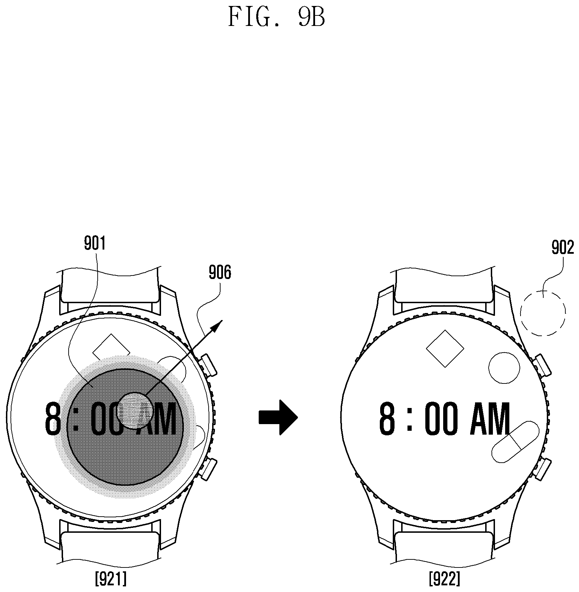

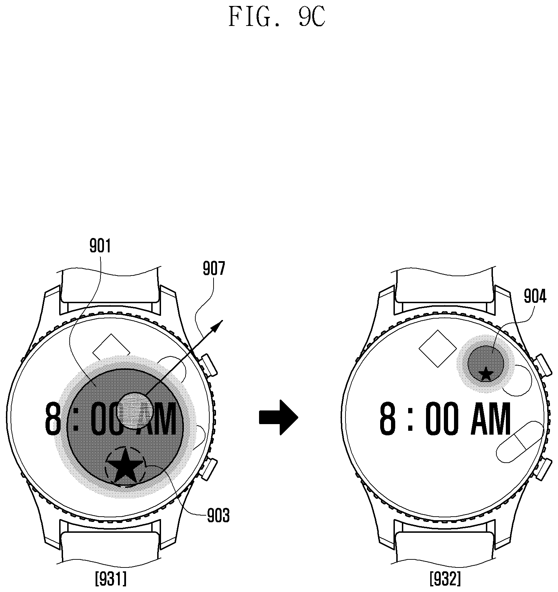

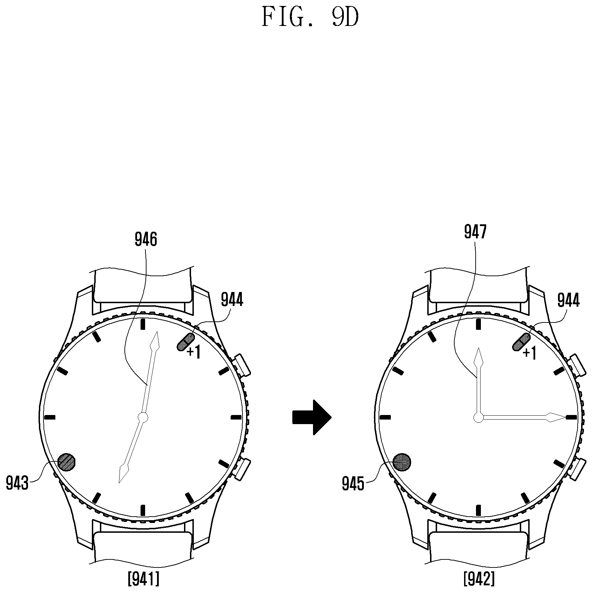

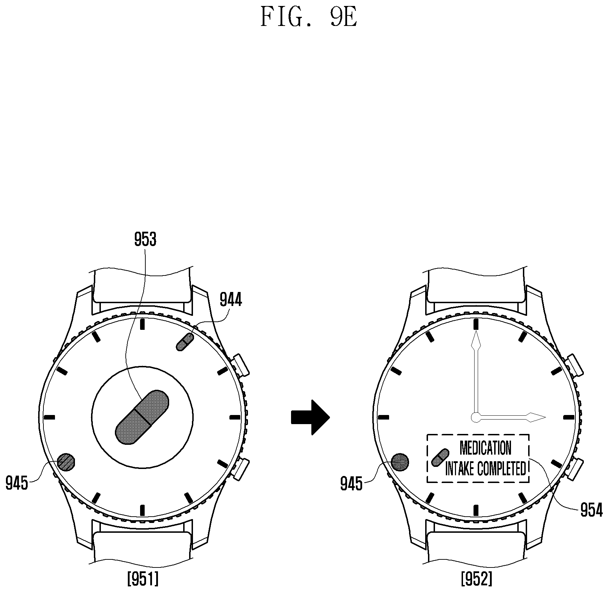

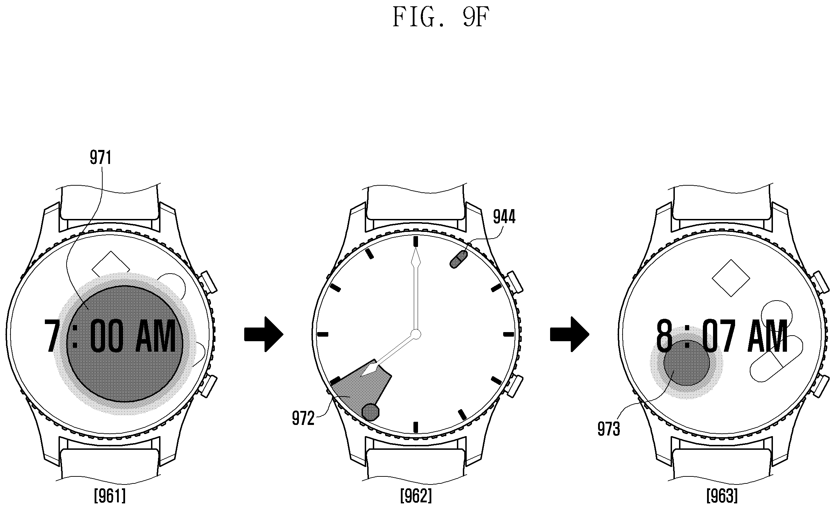

FIG. 9A is a view illustrating an example of displaying a medication icon indicating that medication intake is completed in response to a user input, according to an embodiment of the disclosure. FIG. 9B is a view illustrating an example of displaying a medication icon indicating that medication intake is skipped in response to a user input, according to an embodiment of the disclosure. FIG. 9C is a view illustrating an example of displaying a medication icon indicating important medication intake in response to a user input, according to an embodiment of the disclosure. FIG. 9D is a view illustrating an example in which a user interface changes over time, according to an embodiment of the disclosure. FIG. 9E is a view illustrating an example of displaying a message indicating a state in which medication intake is completed, according to an embodiment of the disclosure. FIG. 9F is a view illustrating an example of displaying a medication icon for medication to be taken in a state where a designated medication intake time has elapsed, according to an embodiment of the disclosure.

The electronic device 101 in FIGS. 9A to 9F may be at least partially similar to the electronic device 101 in FIGS. 1 to 6, or may further include other embodiments of an electronic device.

According to an embodiment, the electronic device 101 may include a wearable electronic device wearable on a part (e.g., a wrist) of a user's body. For example, the electronic device 101 may include a wearable watch worn on the user's wrist. When worn, the electronic device 101 may maintain a state of being at least partially in contact with the user's skin. The electronic device 101 may display at least one medication icon related to medication intake through a display (e.g., the display 220 in FIG. 6), and when a set medication notification time is reached, the electronic device 101 may output a notification signal related to medication intake. The electronic device 101 may apply a visual effect to the medication icon.

Referring to FIG. 9A, a first screen 911 may include a first medication icon 901 to which a visual effect is applied in accordance with a set first time (e.g., 8:00 AM). For example, a processor (e.g., the processor 120 in FIG. 6) of the electronic device 101 may identify the first time set corresponding to the first medication icon 901, and when the first time is reached, the processor 120 may apply and display the visual effect to the first medication icon 901. The processor 120 may detect a first user input 905 (e.g., a double tap input) for the first medication icon 901. For example, the electronic device 101 may be in a state where a double tap input is set in relation to a situation where medication intake is completed. After identifying the first screen 911 and taking the first medication, the user may perform the double tap 905 on the display (e.g., the display 220 in FIG. 5). In response to the first user input 905, the processor 120 may dismiss a notification signal set corresponding to the first medication icon 901.

A second screen 912 may be a screen to which an animation effect 909, in which the size of the first medication icon 901 gradually decreases, is applied in response to the first user input 905 for the first medication icon 901. In response to the first user input 905, the processor 120 may dismiss the notification signal set corresponding to the first medication icon 901, and may change a shape of the first medication icon 901 such that the size of the first medication icon 901 gradually decreases.

Referring to FIG. 9B, a third screen 921 may include the first medication icon 901 to which a visual effect is applied in accordance with the set first time (e.g., 8:00 AM). The processor 120 may detect a second user input 906 (e.g., a drag input) for the first medication icon 901. For example, the second user input 906 may include a drag input in which a touch input for the first medication icon 901 is moved outside the display 220 while the touch input is maintained. For example, the electronic device 101 may be in a state where a drag input moving outside the clock screen (e.g., the display 220) is set in relation to a situation where medication is not taken (e.g., a situation of passing medication).

A fourth screen 922 may be a screen to which an animation effect 902, in which the first medication icon 901 moves outside the clock screen (e.g., the display 220) and disappears from the clock screen, is applied in response to the second user input 906 for the first medication icon 901. In response to the second user input 906, the processor 120 may record that the first medication icon 901 has not been taken, and may adjust the user interface so that the first medication icon 901 is not displayed on the clock screen.

Referring to FIG. 9C, a fifth screen 931 may include the first medication icon 901 to which a visual effect is applied in accordance with the set first time (e.g., 8:00 AM). The processor 120 may detect a third user input 907 (e.g., a drag input) for the first medication icon 901. For example, the third user input 907 may include a drag input in which a touch input for the first medication icon 901 is moved outside the display 220 while the touch input is maintained. The third user input 907 may be substantially the same input as the second user input 906 in FIG. 9B. The first medication icon 901 in FIG. 9C may be set as an essential medication that the user must take, and may include a mark 903 (e.g., an image, a label, or a symbol) corresponding to the essential medication. For example, a medication icon on which the essential medication-related mark 903 is displayed may be set such that medication intake cannot be passed.

A sixth screen 932 may be a screen on which the first medication icon 901 remains on the clock screen (e.g., the display 220) even if the third user input 907 is identified, because the first medication icon 901 is set as an essential medication. Even if the third user input 907 is identified, the processor 120 may adjust the user interface so that the first medication icon 901 (e.g., medication set as an essential medication) is displayed on the clock screen.

Referring to FIG. 9D, a seventh screen 941 may include medication icons 943 and 944 related to at least one medication scheduled for intake today, based on an analog clock screen. For example, the current time is 6:02 AM 946, and the seventh screen 941 may display the medication icons 943 and 944 scheduled for intake for which a medication intake notification time has not elapsed. For example, a second medication icon 943 may be in a state where a medication intake notification time is set at 8:00 AM, and a third medication icon 944 may be in a state where a medication intake notification time is set at 1:00 PM. The medication for which the medication intake notification time is set at 1:00 PM may include an additional medication denoted by an indicator (+1) along with the third medication icon 944. According to an embodiment, the electronic device 101 may display an analog-type clock screen and may display at least one medication icon 943 and 944 scheduled for intake based on the analog-type clock screen.

In the case of an eighth screen 942, the current time is 12:15 PM 947, and medication icons 944 and 945 scheduled for intake for which a medication intake notification time has not elapsed may be displayed. For example, the third medication icon 944 may be in a state where a medication intake notification time is set at 1:00 PM, and a fourth medication icon 945 may be in a state where a medication intake notification time is set at 8:00 PM. Referring to the seventh screen 941 and the eighth screen 942, intake of the second medication corresponding to the second medication icon 943 was completed at 8:00 AM, and the fourth medication icon 945 scheduled to be taken thereafter based on the current time may be displayed.

Referring to FIG. 9E, this may be a diagram illustrating a situation after the eighth screen 942 shown in FIG. 9D. A ninth screen 951 may include a screen on which an image 953 corresponding to the third medication icon 944 is displayed in a state where a medication intake notification time for the third medication icon 944 is reached. The processor 120 may display the image 953 corresponding to the third medication icon 944 in a form that at least partially overlaps the clock screen.

In the case of a tenth screen 952, the current time is 3:00 PM, and an intake completion message 954 indicating that the intake of medication corresponding to the third medication icon 944 is completed may be displayed. For example, the fourth medication icon 945 scheduled for intake, for which the medication intake notification time has not elapsed, may be maintained on the clock screen.

Referring to FIG. 9F, an eleventh screen 961 may include a fifth medication icon 971 to which a visual effect is applied in accordance with a set time (e.g., 7:00 AM). For example, the processor 120 may identify a time set corresponding to the fifth medication icon 971, and when the time is reached, the processor 120 may apply and display a visual effect to the fifth medication icon 971. The eleventh screen 961 may include a digital-type clock screen.

A twelfth screen 962 may include an analog-type clock screen and may include the fifth medication icon for which a medication intake notification time has elapsed. For example, in the case of the twelfth screen 962, the current time is 8:00 AM, and the screen may display a situation where approximately one hour has elapsed from the medication intake notification time (e.g., 7:00 AM) of the fifth medication icon. In the twelfth screen 962, a visual effect may be applied to a time section 972 indicating that the medication intake notification time has elapsed. For example, in response to a situation where the medication intake notification time elapses, the processor 120 may change and display the digital clock screen (e.g., the eleventh screen 961) to the analog clock screen (e.g., the twelfth screen 962), and may change and display a color of the time section 972 indicating that the medication intake notification time has elapsed.

A thirteenth screen 963 may include the fifth medication icon 973 to which a visual effect is applied in a situation where approximately one hour has elapsed from the medication intake notification time. For example, the fifth medication icon 973 may be displayed with an emphasis effect applied thereto so as to be distinguished from the remaining medications.

According to an embodiment, the electronic device 101 may identify a medication intake notification time for at least one medication and may display a medication-related user interface in accordance with a predetermined medication intake notification time so that the user can correctly take medication. The electronic device 101 may apply a visual effect to a medication icon for which a medication intake notification time has elapsed, and may adjust the user interface so as to visually distinguish such a medication from the remaining other medications.

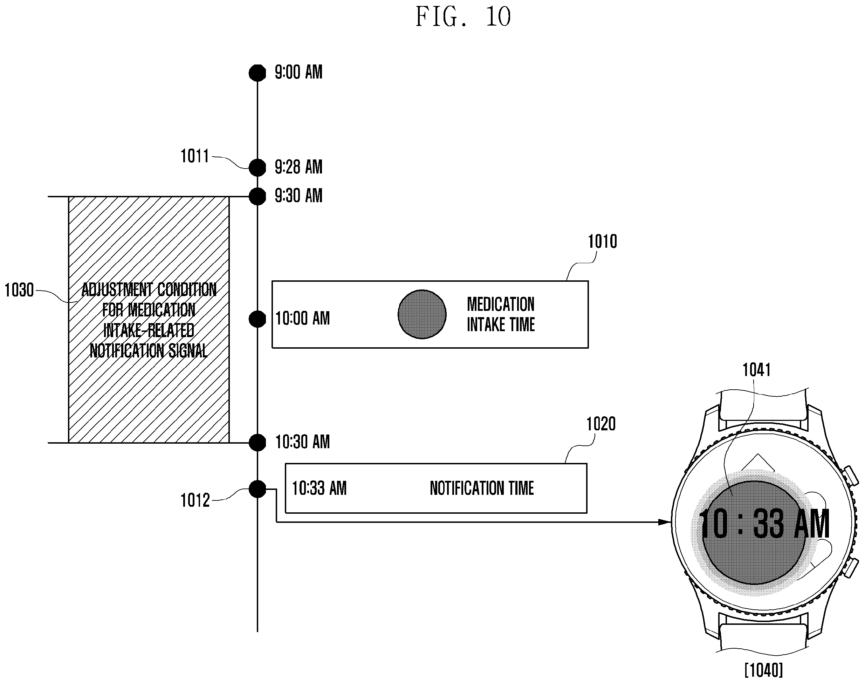

FIG. 10 is a view illustrating an example of adjusting an output timing of a notification signal based on adjustment conditions for a medication intake-related notification signal and outputting a notification signal in which a visual effect is reflected, according to an embodiment of the disclosure.

The electronic device 101 in FIG. 10 may be at least partially similar to the electronic device 101 in FIGS. 1 to 6, or may further include other embodiments of an electronic device.

Referring to FIG. 10, the electronic device 101 may be in a state 1010 where a medication intake notification time corresponding to a first medication is set to 10:00 AM. For example, the user may be in a state where a meeting is scheduled from 9:30 AM to 10:30 AM as a today's schedule. The electronic device 101 may be in a state where a schedule related to the meeting is registered based on a calendar application (e.g., a schedule-related application).

For example, at 9:28 AM, the electronic device 101 may be in a state 1011 of having entered a meeting room. The electronic device 101 may periodically or aperiodically identify a location of the electronic device 101, and may be in a state of having obtained the meeting-related schedule from the calendar application. Referring to FIG. 10, an adjustment condition 1030 for a medication intake-related notification signal may include the meeting time scheduled for today. According to an embodiment, the adjustment condition 1030 for the medication intake-related notification signal may include at least one of biometric information for the user (e.g., heart rate information, body temperature information, or ECG information), location information of the electronic device 101 (e.g., a medication intake location according to a medication intake history), and/or other schedule information (e.g., in a meeting, in travel, or in a conversation). For example, in outputting a medication intake notification signal, the electronic device 101 may identify the adjustment condition 1030 for the medication intake-related notification signal, and may adjust an output timing of the medication intake notification signal based on the adjustment condition 1030 for the medication intake-related notification signal.

For example, the processor 120 of the electronic device 101 may identify the location of the electronic device 101, based on the medication intake notification time, and may compare it with a previous medication intake history to determine whether the user has ever taken medication at the identified location. If the user has never taken medication at the identified location, the processor 120 may adjust the output timing of the medication intake notification signal until the electronic device 101 leaves the identified location. As another example, the processor 120 may identify whether there is another schedule (e.g., a meeting, a conference, or a personal schedule) that at least partially overlaps, based on the medication intake notification time. If another schedule is included in the medication intake notification time, the processor 120 may adjust the output timing of the medication intake notification signal until the other schedule is completed. As another example, the processor 120 may obtain biometric information of the user, based on the medication intake notification time, and may determine whether the user is in a state capable of taking medication, based on the obtained biometric information. For example, if it is identified that the user's heart rate exceeds a set heart rate, the user may be in a situation where taking medication is difficult at the current time. If the user's heart rate exceeds the set heart rate, the processor 120 may adjust the output timing of the medication intake notification signal until the user's heart rate returns to within a normal range (e.g., below the set heart rate).

Referring to FIG. 10, the processor 120 may identify a state 1012 in which the electronic device 101 leaves the meeting room at 10:33 AM. In response to identifying the state 1011 in which the electronic device 101 leaves the meeting room, the processor 120 may output a medication intake notification signal 1020. For example, upon outputting the medication intake notification signal 1020, the electronic device 101 may apply and display a visual effect to a first medication icon 1041 corresponding to the first medication in a first screen 1040.

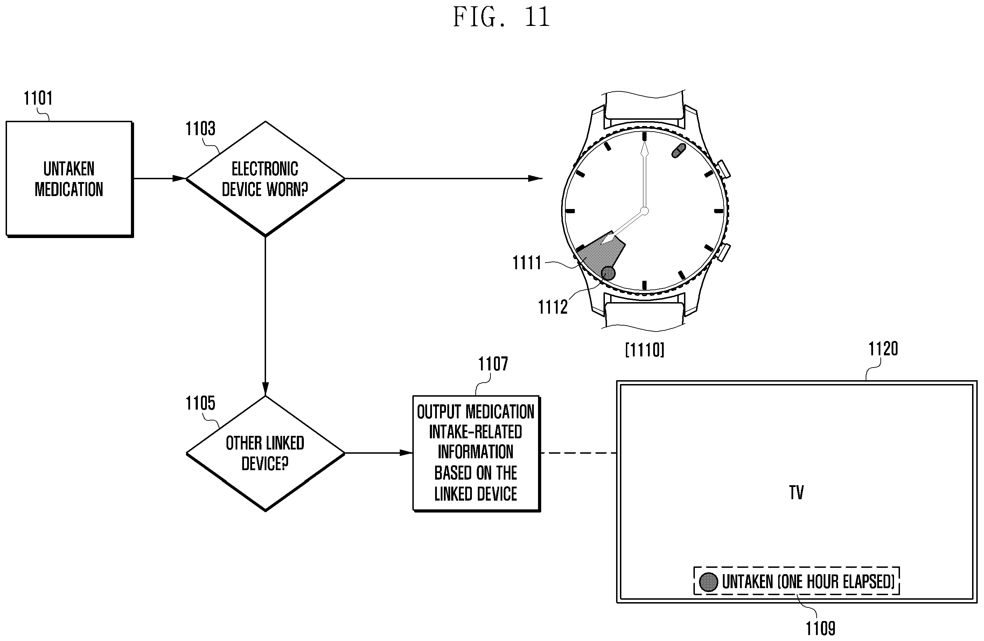

FIG. 11 is a view illustrating an example of outputting a medication intake-related notification signal via another electronic device linked with the electronic device in a state where the electronic device is not worn, according to an embodiment of the disclosure.

The electronic device 101 in FIG. 11 may be at least partially similar to the electronic device 101 in FIGS. 1 to 6, or may further include other embodiments of an electronic device.

Referring to FIG. 11, the electronic device 101 may identify an untaken medication (e.g., missed medication), based on a set medication intake notification time, and may provide a notification signal related to medication intake to a user. In operation 1101, the processor 120 of the electronic device 101 may identify that there is an untaken medication, and in operation 1103, the processor 120 may determine whether the electronic device 101 is being worn on a part (e.g., a wrist) of the user's body. For example, using a wearing detection sensor (e.g., the wearing detection sensor 624 in FIG. 6), the processor 120 may identify whether the electronic device 101 maintains a state of being in physical contact with the user's wrist.

For example, when the electronic device 101 is in a state of being worn on the user's wrist, the processor 120 may display a medication icon 1112 corresponding to the untaken medication on a first screen 1110, and may visually display a time section 1111 elapsed from the medication intake notification time.

For example, when the electronic device 101 is in a state of not being worn on the user's wrist, the processor 120 may determine in operation 1105 whether there is another device operatively linked with the electronic device 101. For example, the electronic device 101 may be in a state of being communicatively connected to another external electronic device through a communication circuit (e.g., the communication circuit 690 in FIG. 6). If another device linked with the electronic device 101 exists in operation 1105, the processor 120 may output medication intake-related information based on the linked device in operation 1107. For example, if the linked device is a television (TV) 1120, the electronic device 101 may transmit a command signal for outputting a notification message 1109 to the television 1120, and may at least partially control the operation of the television 1120 such that the notification message 1109 is output through a screen of the television 1120.

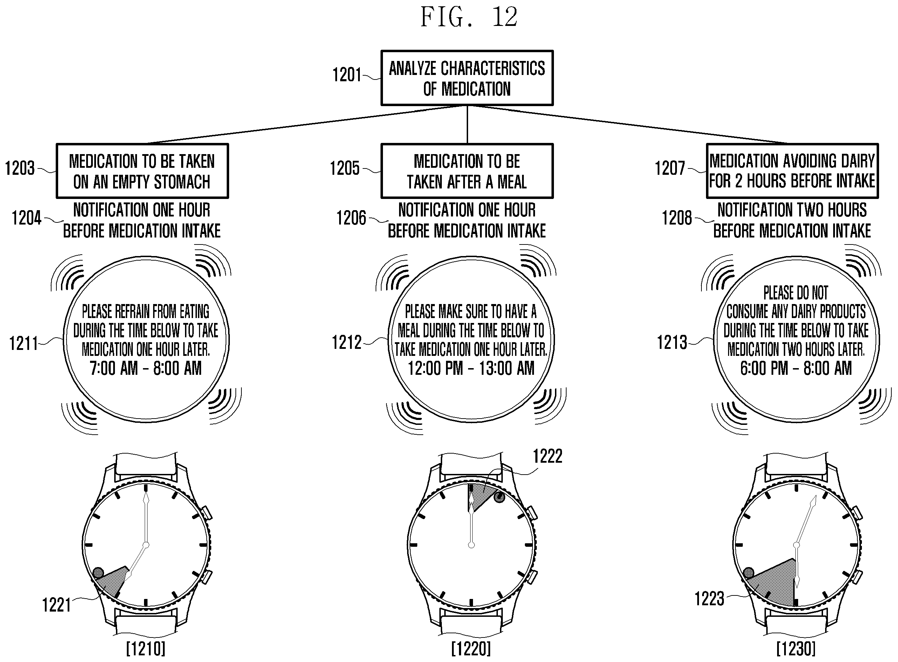

FIG. 12 is a view illustrating an example of displaying a notification message based on medication-related information and displaying a user interface in which a visual effect associated with the medication-related information is reflected, according to an embodiment of the disclosure.

The electronic device 101 in FIG. 12 may be at least partially similar to the electronic device 101 in FIGS. 1 to 6, or may further include other embodiments of an electronic device.

Referring to FIG. 12, the electronic device 101 may identify characteristics and an intake method of a medication in relation to the medication to be taken. For example, the electronic device 101 may be operatively connected for communication with an external electronic device, and may obtain various types of medication-related information (e.g., ingredients, intake methods, effects, intake frequency, precautions, correlation with other medications, side effects, images, and/or shapes) from the external electronic device. For example, the external electronic device may include a server that manages various types of medication-related information in a database (DB).

In operation 1201, the processor 120 of the electronic device 101 may analyze the characteristics of the medication to be taken. For example, in operation 1203, the processor 120 may identify a medication to be taken on an empty stomach, and in operation 1204, the processor 120 may output a notification message to refrain from food intake approximately one hour before a medication intake notification time. As another example, in operation 1205, the processor 120 may identify a medication to be taken after a meal, and in operation 1206, the processor 120 may output a notification message recommending food intake approximately one hour before the medication intake notification time. As another example, in operation 1207, the processor 120 may identify a medication for which intake of dairy products is prohibited two hours before medication intake, and in operation 1208, the processor 120 may output a notification message to refrain from intake of dairy products approximately two hours before the medication intake notification time.

After operations 1203 and 1204, the processor 120 may display a first notification message 1211 to refrain from food intake through a display (e.g., the display 220 in FIG. 6). As shown in a first screen 1210, the processor 120 may apply and display a visual effect to a one-hour time section 1221 prior to the medication intake notification time.

After operations 1205 and 1206, the processor 120 may display a second notification message 1212 recommending food intake through the display 220. As shown in a second screen 1220, the processor 120 may apply and display a visual effect to a one-hour time section 1222 prior to the medication intake notification time.

After operations 1207 and 1208, the processor 120 may display a third notification message 1213 regarding the medication for which intake of dairy products is prohibited through the display 220. As shown in a third screen 1230, the processor 120 may apply and display a visual effect to a two-hour time section 1223 prior to the medication intake notification time.

According to an embodiment, the electronic device 101 may identify characteristics and an intake method related to at least one medication scheduled for intake, and based on the identified characteristics and intake method, may display a medication-related notification message so that a user can correctly take the medication. The electronic device 101 may apply and display a visual effect for the medication to be taken so that the user can intuitively identify the information.

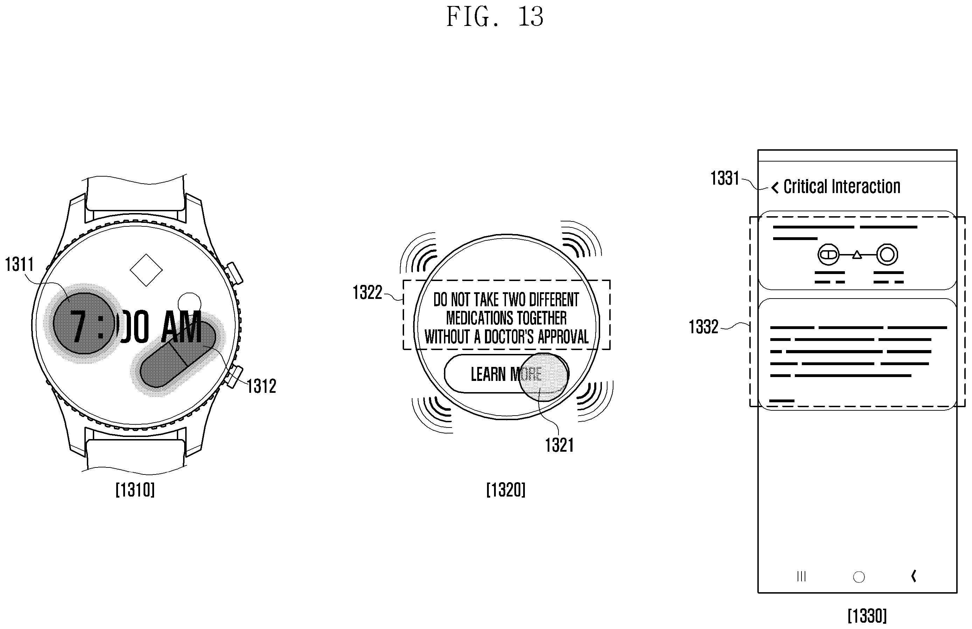

FIG. 13 is a view illustrating an example of displaying a notification message based on medication-related information and displaying medical information related to medication intake, according to an embodiment of the disclosure.

The electronic device 101 in FIG. 13 may be at least partially similar to the electronic device 101 in FIGS. 1 to 6, or may further include other embodiments of an electronic device.

Referring to FIG. 13, the electronic device 101 may identify medical information and precautions for use in relation to a medication to be taken. For example, the electronic device 101 may obtain medical information and precautions for use from an external electronic device (e.g., a server), and based on this, may output a medication intake notification signal.

In a first screen 1310 of FIG. 13, the processor 120 of the electronic device 101 may apply and display a visual effect to medication icons 1311 and 1312 scheduled for intake in accordance with a set medication intake notification time. For example, when medical opinions and precautions are identified in relation to the medication scheduled for intake, the processor 120 may apply a visual effect different from a visual effect for a general medication icon. For example, by applying a different color to the medication icon, it may be indicated that the medication scheduled for intake is a medication requiring precautions.

In a second screen 1320, the processor 120 may display a notification message 1322 for medication intake and may provide an option for identifying medical information related to the medication intake. In response to a user input 1321 for the option displayed on the second screen 1320, the electronic device 101 may provide the medical information of a third screen 1330 to the user. For example, the electronic device 101 may output the medical information through a portable terminal device (e.g., a smartphone) operatively connected for communication. Although not shown, the medical information may also be output through a display (e.g., the display 220 in FIG. 6) of the electronic device 101. For example, the medical information may include detailed information 1331 and 1332 that should be noted when taking the medication.

According to an embodiment, the electronic device 101 may classify precautions in stages in relation to the medication to be taken, and may apply different visual effects for each precaution. For example, a medication for which a doctor's prescription is essential may be displayed by applying a first effect implemented centered on a red color, and a medication for which a doctor's prescription is recommended may be displayed by applying a second effect implemented centered on a yellow color.

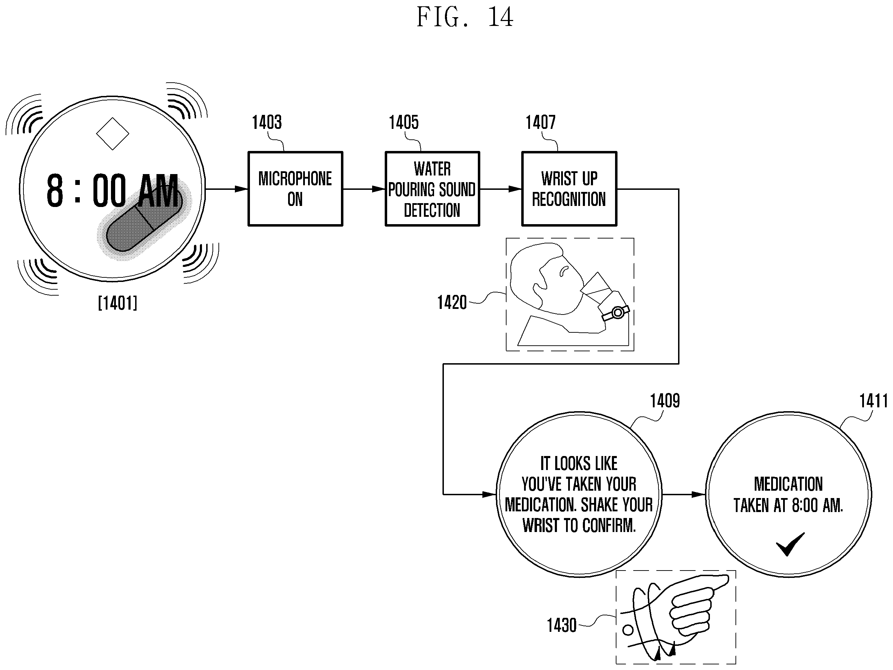

FIG. 14 is a view illustrating an example of detecting a movement of an electronic device caused by medication intake and recognizing completion of medication intake based on the detected movement, according to an embodiment of the disclosure.

The electronic device 101 in FIG. 14 may be at least partially similar to the electronic device 101 in FIGS. 1 to 6, or may further include other embodiments of an electronic device.

Referring to FIG. 14, the electronic device 101 may output a medication intake notification signal, as shown in a first screen 1401, based on a set medication intake notification time (e.g., 8:00 AM). In response to the output of the medication intake notification signal, in operation 1403, the electronic device 101 may at least partially activate a microphone (e.g., the microphone 650 in FIG. 6) and obtain an external audio signal using the microphone 650. In operation 1405, the electronic device 101 may detect a sound of pouring water based on the obtained audio signal. For example, the electronic device 101 may detect the sound of the user pouring water to take medication within a set time after outputting the medication intake notification signal. In operation 1407, the electronic device 101 may at least partially activate a motion sensor (e.g., the motion sensor 621 in FIG. 6) and recognize a movement of the electronic device 101 using the motion sensor 621. For example, the electronic device 101 may recognize a gesture 1420 of the user taking medication (e.g., a gesture of putting medication into the mouth or a gesture of drinking water). The electronic device 101 is in a state of being worn on the user's wrist, and may recognize the gesture 1420 of the user taking medication based on the position and orientation of the user's wrist.

In operation 1409, the electronic device 101 may display a notification message 1409 for confirming whether the intake of the medication to be taken is completed. For example, although the electronic device 101 may determine whether the medication has been taken based on the audio signal including the sound of pouring water and the gesture of taking medication, the electronic device 101 may provide the notification message 1409 (e.g., a notification message to perform a predetermined gesture if intake is completed) to the user for accurate confirmation.

For example, the user may perform a gesture 1430 (e.g., a wrist-shaking motion) described in the notification message 1409. The electronic device 101 may recognize the user's gesture 1430, and in operation 1411, may determine that the intake of the medication is completed. In operation 1411, the electronic device 101 may dismiss the medication intake notification signal related to the medication intake. The electronic device 101 may record that the medication intake has been correctly completed.

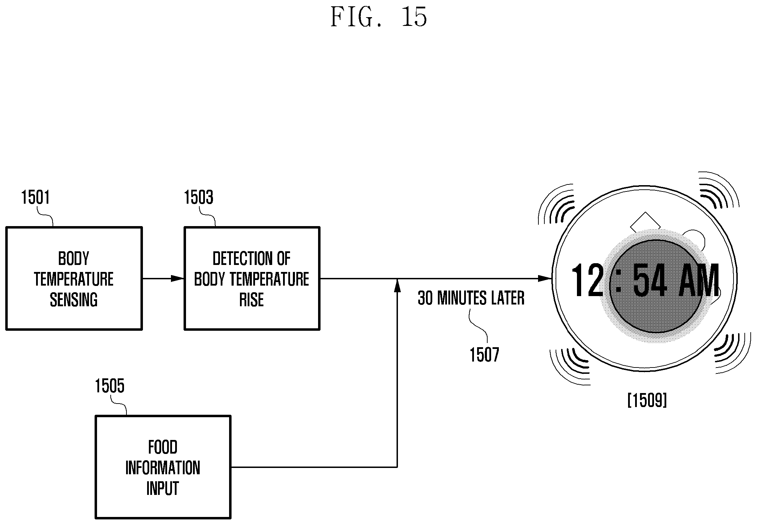

FIG. 15 is a view illustrating an example of identifying a user's body temperature and adjusting an output timing of a notification signal based on the identified body temperature, according to an embodiment of the disclosure.

The electronic device 101 in FIG. 15 may be at least partially similar to the electronic device 101 in FIGS. 1 to 6, or may further include other embodiments of an electronic device.

Referring to FIG. 15, in operation 1501, the processor 120 of the electronic device 101 may at least partially activate a body temperature measurement sensor (e.g., the body temperature measurement sensor 622 in FIG. 6), based on a set medication intake notification time, and may measure a user's body temperature using the activated body temperature measurement sensor 622.

In operation 1503, the processor 120 may compare the measured body temperature with an average body temperature of the user, and when the measured body temperature has risen above a set value, the processor 120 may postpone the medication intake notification time to approximately 30 minutes later. For example, an increase in body temperature may mean that the user is exercising or is in a physically excited state, which may be a situation in which it is difficult to take medication.

In operation 1507, the processor 120 may adjust the medication intake notification time to be 30 minutes later, and after the medication intake notification time has elapsed by approximately 30 minutes, as shown in a first screen 1509, the processor 120 may output a medication intake notification signal at 12:54 PM.

Referring to FIG. 15, in operation 1505, when food information is input based on a health-related application (e.g., a health application or a food management application), in operation 1507, the processor 120 may postpone the medication intake notification time to approximately 30 minutes later. For example, if it is identified that the user is having a meal in operation 1505, the processor 120 may adjust an output timing of the medication intake notification signal so that the medication intake notification signal is postponed by a set time.

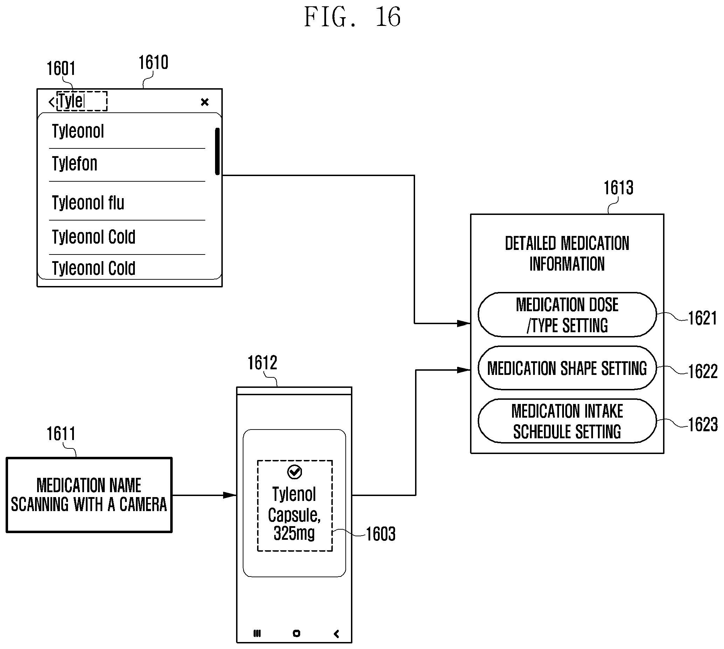

FIG. 16 is a view illustrating an example of registering a medication by searching for a medication name and registering a medication by scanning a medication listed on a prescription with a camera, according to an embodiment of the disclosure.

The operations shown in FIG. 16 may be performed by an electronic device (e.g., the electronic device 101 in FIGS. 1 to 6) or may be performed by another electronic device (e.g., a portable terminal device or a smartphone) operatively linked with the electronic device 101. The screen shown in FIG. 16 is not limited to a rectangular shape. The electronic device 101 in FIG. 16 may be at least partially similar to the electronic device 101 in FIGS. 1 to 6, or may further include other embodiments of an electronic device.

Referring to FIG. 16, the electronic device 101 may provide a user with various methods for registering a medication to be taken. According to an embodiment, the processor 120 of the electronic device 101 may display a first user interface 1610 including a search area 1601 used to search for a medication, and may search for at least one medication based on text information (e.g., text information input by the user) input into the search area 1601. According to another embodiment, in response to execution of a medication search function, the processor 120 may activate a camera (e.g., the camera module 180 in FIG. 1) and search for at least one medication using a scanning function through the camera 180.

According to an embodiment, the processor 120 may register detailed information related to the searched medication (e.g., a medication dose/type setting 1621, a medication shape setting 1622, and/or a medication intake schedule setting 1623) in medication intake schedule information (e.g., the medication intake schedule information 611 in FIG. 6).

In operation 1601, the processor 120 may identify the execution of a function related to a medication search and may display the search area 1601 used for inputting medication information to be searched. The processor 120 may display the first user interface 1610 including the search area 1601. For example, the processor 120 may output at least one medication, based on the text information input into the search area 1601. The processor 120 may receive information on the at least one medication based on a medication-related database (DB) of an external electronic device (e.g., a server), and may display the at least one medication corresponding to the text information in the form of a list. For example, when one medication for registration is selected, in operation 1613, the processor 120 may set detailed information for the selected medication. The processor 120 may provide a first option 1621 for setting a medication dose and a medication type (e.g., a pill, powder, and/or liquid), a second option 1622 for setting a medication shape, and/or a third option 1623 for setting a medication intake schedule. For example, the processor 120 may output a pop-up window for setting detailed information in a form that at least partially overlaps the first user interface 1610.

In operation 1611, the processor 120 may identify the execution of a function related to a medication scan, and may scan a medication name by activating the camera 180. For example, when there is a medication prescription, the electronic device 101 may scan medication information (e.g., a medication name, a medication type, the number of medications, or a medication intake time) described in the medication prescription. The processor 120 may display a second user interface 1612 including scanning result data 1603. The processor 120 may receive information on the at least one medication based on a medication-related database (DB) of an external electronic device (e.g., a server), and may determine the scanning result data 1603 based on the received information on the at least one medication. When the scanning result data 1603 is determined, the processor 120 may register the medication intake schedule information 611 related to the at least one medication or display a user interface (e.g., a menu) for the registration. For example, when a user input for registration (e.g., an input for entering a next step after scanning) is detected, the processor 120 may provide a menu for setting detailed information corresponding to the one medication selected in operation 1613.

According to an embodiment, the electronic device 101 provides various methods for registering medication to be taken (i.e., medication intake). The electronic device 101 may provide a medication registration service to a user, based on a partner (e.g., an application or a medication intake management application) linked with a prescription. For example, when the prescription includes code information (e.g., a quick response (QR) code, encrypted code information related to medication to be taken, or code information including medication intake information) for executing the medication registration service, the processor 120 of the electronic device 101 may activate the camera 180 and capture (e.g., scan) the code information using the activated camera 180. Upon scanning the code information, the electronic device 101 may execute the medication intake management application, obtain medication information to be taken (e.g., medication intake information) according to the scanning, based on the medication intake management application, and register the medication information in the medication intake schedule information 611. For example, with a single scan of the code information, the electronic device 101 may register and record a schedule for medication to be taken. The medication intake management application may manage the medication intake schedule information 611 and may output a notification signal in accordance with the schedule for the medication to be taken. According to an embodiment, the medication registration method is not limited to the aforementioned method, and schedule information for at least one medication may be registered based on various methods supported by the medication intake management application.

According to an embodiment, the electronic device 101 may provide information on food items that interact with the medication to be taken, based on a partner (e.g., an application or a medication intake management application) linked with the prescription. For example, the medication intake management application may display luxury foods (e.g., cigarettes, alcohol, tea, candy, or chocolate) and food items that affect the human body when consumed with the medication to be taken. The electronic device 101 may provide the user with various types of information existing in association with the medication to be taken. For example, the electronic device 101 may obtain various types of information associated with at least one medication, based on a medication-related database (DB) of a server, and may provide the obtained information to the user. The electronic device 101 may display the various types of information associated with the at least one medication through a display, based on the medication intake management application.

According to an embodiment, upon registering the medication to be taken, the electronic device 101 may identify a medication dose and a medication type based on the medication-related database (DB) of the server, and may display a plurality of menus selectable by the user.

A method for outputting a notification signal for medication intake in an electronic device according to an embodiment may include: displaying a clock screen including an icon for at least one medication based on medication intake schedule information 611; identifying a notification time for taking the at least one medication based on the medication intake schedule information 611; identifying an adjustment condition for a medication intake-related notification signal based on the identified notification time; adjusting an output timing of a notification signal for taking the at least one medication based on the identified adjustment condition for the medication intake-related notification signal; and displaying a visual effect corresponding to the icon included in the clock screen based on the adjusted output timing.

The method according to an embodiment may further include: in response to the output of the notification signal, executing a recording function for a microphone 650; determining whether the at least one medication has been taken, based on at least one of an audio signal obtained using the recording function or gesture-related information obtained based on a motion sensor 621; and in response to confirming that the at least one medication has been taken, dismissing a notification output function according to the notification time.

The method according to an embodiment may further include: in response to confirming that the at least one medication has been taken based on at least one of the audio signal or the gesture-related information, displaying a medication intake-related notification message; and in response to a user input for the notification message, changing a parameter value indicating whether the at least one medication has been taken.

According to an embodiment, the visual effect corresponding to the icon may include, for the icon, a change in size, a change in color, a change in shape, an emphasis effect, and a display of precautions related to medication.

According to an embodiment, the adjustment condition for the medication intake-related notification signal may determine whether to adjust an output timing of the medication intake-related notification signal, based on at least one of biometric information (e.g., heart rate, body temperature, or ECG) by which one of an exercise state and a sleeping state is identified, location information of the electronic device 101 (e.g., a medication intake location identified according to a medication intake history), and other schedule information (e.g., during a meeting, during travel, or during a conversation).

The method according to an embodiment may further include: identifying that the user is in an exercise state, based on at least one of body temperature information measured using a body temperature measurement sensor 622 and heart rate information measured using a heart rate measurement sensor 623; and in response to identifying the exercise state, adjusting the output timing of the notification signal for taking the at least one medication.

The method according to an embodiment may further include: identifying heart rate information by the heart rate measurement sensor 623 and movement information by the motion sensor 621; identifying that the user is in a sleeping state, based on the heart rate information and the movement information; and in response to identifying the sleeping state, adjusting the output timing of the notification signal for taking the at least one medication.

The method according to an embodiment may further include: determining whether the electronic device 101 is in a state of being worn on a part of the user's body, using a wearing detection sensor 624; in response to the electronic device 101 being in an unworn state, identifying other electronic device 102 or 104 operatively connected to the electronic device 101 through a communication circuit 390; and outputting a notification signal related to taking the at least one medication, based on the identified other electronic device 102 or 104.

The method according to an embodiment may further include: receiving medication-related information from an external electronic device 108; and displaying precautions related to taking the at least one medication through a display 220, based on the received medication-related information.

The method according to an embodiment may further include: when a user's activity is identified based on information (e.g., schedule information) received from other application (e.g., a calendar application or a schedule-related application), adjusting the output timing of the notification signal for taking the at least one medication based on the information received from the other application.

According to an embodiment, a non-transitory computer-readable storage medium (or a computer program product) that stores one or more programs may be described. According to an embodiment, the one or more programs may include instructions that, when executed by a processor of an electronic device, perform operations of: displaying a clock screen including an icon for at least one medication based on medication intake schedule information 611; identifying a notification time for taking the at least one medication based on the medication intake schedule information 611; identifying an adjustment condition for a medication intake-related notification signal based on the identified notification time; adjusting an output timing of a notification signal for taking the at least one medication based on the identified adjustment condition for the medication intake-related notification signal; and displaying a visual effect corresponding to the icon included in the clock screen based on the adjusted output timing.

The electronic device according to various embodiments may be one of various types of electronic devices. The electronic devices may include, for example, a portable communication device (e.g., a smartphone), a computer device, a portable multimedia device, a portable medical device, a camera, a wearable device, a home appliance, or the like. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. It is intended that features described with respect to separate embodiments, or features recited in separate claims, may be combined unless such a combination is explicitly specified as being excluded or such features are incompatible. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), the element may be coupled with the other element directly (e.g., wiredly), wirelessly, or via a third element.

As used in connection with various embodiments of the disclosure, the term "module" may include a unit implemented in hardware, software, or firmware, or any combination thereof, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the "non-transitory" storage medium is a tangible device, and may not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (101) comprising:
a display (220);
a processor (120); and
a memory (130) storing medication intake schedule information (611) and instructions,
wherein the instructions cause, when executed by the processor (120), the electronic device (101) to:
display a clock screen including an icon for at least one medication, based on the medication intake schedule information (611),
identify a notification time for taking the at least one medication, based on the medication intake schedule information (611),
identify an adjustment condition for a medication intake-related notification signal, based on the identified notification time,
adjust an output timing of the notification signal for taking the at least one medication, based on the identified adjustment condition for the medication intake-related notification signal, and
display a visual effect corresponding to the icon included in the clock screen, based on the adjusted output timing.

2. The electronic device of claim 1, further comprising:
a motion sensor (621); and
a microphone (650),
wherein the instructions cause, when executed by the processor (120), the electronic device (101) to:
execute a recording function for the microphone (650) in response to an output of the notification signal,
determine whether the at least one medication has been taken, based on at least one of an audio signal obtained using the recording function or gesture-related information obtained based on the motion sensor (621), and
dismiss a notification output function according to the notification time in response to confirming that the at least one medication has been taken.

3. The electronic device of claim 2, wherein the instructions cause, when executed by the processor (120), the electronic device (101) to:
display a medication intake-related notification message in response to confirming that the at least one medication has been taken based on at least one of the audio signal or the gesture-related information, and
change a parameter value indicating whether the at least one medication has been taken, in response to a user input for the notification message.

4. The electronic device of claim 1, wherein the visual effect corresponding to the icon includes, for the icon, a change in size, a change in color, a change in shape, an emphasis effect, and a display of precautions related to medication.

5. The electronic device of claim 2, wherein the adjustment condition for the medication intake-related notification signal determines whether to adjust the output timing of the medication intake-related notification signal, based on at least one of biometric information by which one of an exercise state and a sleeping state is identified, location information of the electronic device (101), and other schedule information.

6. The electronic device of claim 5, further comprising:
a body temperature measurement sensor (622) for measuring a user's body temperature; and
a heart rate measurement sensor (623) for measuring a user's heart rate,
wherein the instructions cause, when executed by the processor (120), the electronic device (101) to:
identify that the user is in the exercise state, based on at least one of body temperature information measured using the body temperature measurement sensor (622) and heart rate information measured using the heart rate measurement sensor (623), and
adjust the output timing of the notification signal for taking the at least one medication, in response to identifying the exercise state.

7. The electronic device of claim 6, wherein the instructions cause, when executed by the processor (120), the electronic device 101 to:
identify heart rate information by the heart rate measurement sensor (623) and movement information of the electronic device (101) by the motion sensor (621),
identify that the user is in the sleeping state, based on the heart rate information and the movement information, and
adjust the output timing of the notification signal for taking the at least one medication, in response to identifying the sleeping state.

8. The electronic device of claim 1, further comprising:
a wearing detection sensor (624); and
a communication circuit (690),
wherein the instructions cause, when executed by the processor (120), the electronic device (101) to:
determine whether the electronic device (101) is in a state of being worn on a part of a user's body, using the wearing detection sensor (624),
identify other electronic device (102, 104) operatively connected to the electronic device (101) through the communication circuit (690), in response to the electronic device (101) being in an unworn state, and
output a notification signal related to taking the at least one medication, based on the identified other electronic device (102, 104).

9. The electronic device of claim 1, wherein the instructions cause, when executed by the processor (120), the electronic device (101) to:
receive medication-related information from an external electronic device (108), and
display precautions related to taking the at least one medication through the display (220), based on the received medication-related information.

10. The electronic device of claim 1, wherein the instructions cause, when executed by the processor (120), the electronic device (101) to:
when a user's activity is identified based on information received from other application, adjust the output timing of the notification signal for taking the at least one medication based on the information received from the other application.

11. The electronic device of claim 1, further comprising:
a camera (180),
wherein the instructions cause, when executed by the processor (120), the electronic device (101) to:
scan medication intake information related to the at least one medication, using the camera (180), and
register the medication intake schedule information (611) corresponding to the at least one medication, based on the scanned medication intake information.

12. A method for outputting a notification signal for medication intake in an electronic device, the method comprising:
displaying a clock screen including an icon for at least one medication, based on medication intake schedule information (611);
identifying a notification time for taking the at least one medication, based on the medication intake schedule information (611);
identifying an adjustment condition for a medication intake-related notification signal, based on the identified notification time;
adjusting an output timing of the notification signal for taking the at least one medication, based on the identified adjustment condition for the medication intake-related notification signal; and
displaying a visual effect corresponding to the icon included in the clock screen, based on the adjusted output timing.

13. The method of claim 12, further comprising:
in response to an output of the notification signal, executing a recording function for a microphone (650);
determining whether the at least one medication has been taken, based on at least one of an audio signal obtained using the recording function or gesture-related information obtained based on a motion sensor (621); and
in response to confirming that the at least one medication has been taken, dismissing a notification output function according to the notification time.

14. The method of claim 13, further comprising:
in response to confirming that the at least one medication has been taken based on at least one of the audio signal or the gesture-related information, displaying a medication intake-related notification message; and
in response to a user input for the notification message, changing a parameter value indicating whether the at least one medication has been taken.

15. A non-transitory computer-readable storage medium storing one or more programs for performing a method of outputting a notification signal for medication intake, the one or more programs comprising:
instructions that, when executed by a processor (120) of an electronic device (101), perform operations of:
displaying a clock screen including an icon for at least one medication, based on medication intake schedule information (611);
identifying a notification time for taking the at least one medication, based on the medication intake schedule information (611);
identifying an adjustment condition for a medication intake-related notification signal, based on the identified notification time;
adjusting an output timing of the notification signal for taking the at least one medication, based on the identified adjustment condition for the medication intake-related notification signal; and
displaying a visual effect corresponding to the icon included in the clock screen, based on the adjusted output timing.
